# EUROPÄISCHE PATENTSCHRIFT

(11) **EP 2 152 711 B1**
(45) Veröffentlichungstag und Bekanntmachung des Hinweises auf die Patenterteilung: **24.11.2010**
(21) Anmeldenummer: 08735269.6
(22) Anmeldetag: 16.04.2008
(51) Int. Cl.: C07D 487/04, A61K 31/407

(54) **2 -HETEROARYL- PYRROLO [3, 4-C]PYRROL- DERIVATE UND DEREN VERWENDUNG ALS SCD INHIBITOREN**
2 -HETEROARYL- PYRROLO [3, 4-C]PYRROLE DERIVATIVES AND THEIR USE AS SCD INHIBITORS
DÉRIVÉS DE 2 -HÉTÉROARYL- PYRROLO [3, 4-C]PYRROLE ET UTILISATION COMME INHIBITEURS DE SCD

(30) Priorität: 27.04.2007 EP 07008646
(43) Veröffentlichungstag der Anmeldung: 17.02.2010
(73) Patentinhaber: Sanofi-Aventis, 75013 Paris (FR)
(72) Erfinder: ZOLLER, Gerhard, 65926 Frankfurt am Main (DE); VOSS, Marc, Dietrich, 65926 Frankfurt am Main (DE); KEIL, Stefanie, 65926 Frankfurt am Main (DE); HERLING, Andreas, 65926 Frankfurt am Main (DE); MATTER, Hans, 65926 Frankfurt am Main (DE)
(74) Vertreter: Fischer, Hans-Jürgen
(86) Internationale Anmeldenummer: PCT/EP2008/003019
(87) Internationale Veröffentlichungsnummer: WO 2008/135141

(56) Entgegenhaltungen:
- WO-A-2006/034315
- WO-A-2006/101521

## Beschreibung

Die Erfindung betrifft Heterocyclische Derivate und deren physiologisch verträgliche Salze.

Es sind bereits strukturähnliche Verbindungen sowie deren Verwendung als SCD 1 Inhibitoren beschrieben. WO2006/03431 A und WO2006/101521 A offenbaren 2-Heteroaryl-hexahydropyrrolo[3,4-c] pyrrolderivate mit SCD inhibierender Wirkung.

Der Erfindung lag die Aufgabe zugrunde, neue Verbindungen zur Verfügung zu stellen, die eine therapeutisch verwertbare Wirkung entfalten. Insbesondere bestand die Aufgabe darin, neue Verbindungen zu finden, die zur Behandlung von erhöhten Lipidkonzentrationen im Blut und in Geweben, dem metabolischen Syndrom, Obesitas, insbesondere viscerale (abdominale) Adipositas, einschließlich der Verhinderung der damit verbundenen Folgeerkrankungen, Diabetes, Insulinresistenz, der Dysregulation von LDL, HLD und VLDL oder Herzkreislauferkrankungen geeignet sind.

Die Erfindung betrifft daher Verbindungen der Formel I, 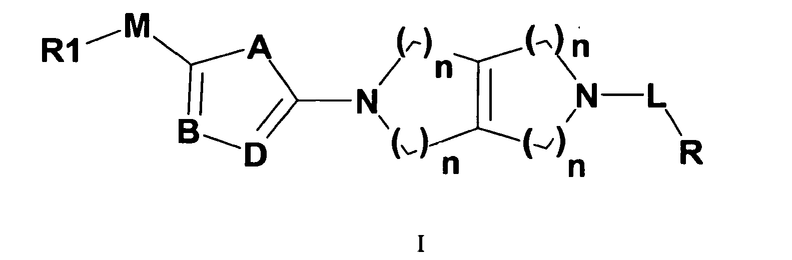 worin bedeuten
- R: Wasserstoff, (C₁-C₁₆)-Alkyl, (C₁-C₅)-Alkyloxy, (C₁-C₅)-Alkylthio, (C₁-C₅)- Alkylamino, Di-(C₂-C₈)-alkylamino, (C₀-C₄)-Alkylen-(C₆-C₁₀)-aryl, (C₀-C₄)- Alkylen-(C₅-C₁₂)-heteroaryl, (C₀-C₄)-Alkylen-(C₃-C₁₂)-heterocyclyl, (C₀-C₄)- Alkylen-(C₃-C₁₂)-cycloalkyl, ein bicyclisches (C₈-C₁₄)-Ringsystem, wobei Aryl, Heteroaryl, Heterocyclyl Cycloalkyl oder das bicyclisches (C₈-C₁₄)-Ringsystem, ein- oder mehrfach mit Halogen, (C₁-C₆)-Alkyl, (C₁-C₃)-Alkyloxy, Hydroxy, (C₁-C₆)-Alkylmercapto, Amino, (C₁-C₆)- Alkylamino, Di-(C₂-C₁₂)-alkylamino, Mono-(C₁-C₆)-alkylaminocarbonyl, Di-(C₂-C₈)-alkylaminocarbonyl, (C₁-C₆)-Alkoxycarbonyl, (C₁-C₆)- Alkylcarbonyl, Cyano, Trifluormethyl, Trifluormethyloxy, (C₁-C₆)- Alkylsulfonyl oder Aminosulfonyl substituiert sein können;
- R1: Wasserstoff, (C₁-C₁₀)-Alkyl, (C₁-C₁₀)-Alkyloxy, Amino, Mono-(C₁-C₁₀)- Alkylamino, Di-(C₂-C₁₂)-alkylamino, wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₅-C₁₂)- Heteroaryl, -(C₃-C₁₂)-Heterocyclyl oder -(C₃-C₁₂)-Cycloalkyl, wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
- R2: Wasserstoff, (C₁-C₁₆)-Alkyl, (C₀-C₄)-Alkylen-(C₆-C₁₀)-aryl;
- R3: Wasserstoff, (C₁-C₆)-Alkyl, (C₁-C₃)-Alkyloxy, Hydroxy, (C₁-C₆)-Alkylmercapto, Amino, (C₁-C₆)-Alkylamino, Di-(C₂-C₁₂)-alkylamino, Cyano, (C₁-C₆)- Alkylcarbonyl, Halogen, Trifluonnethyl, Trifluormethyloxy, (C₁-C₆)- Alkylsulfonyl, Aminosulfonyl;
- A: O, S, N(R2), C(R3), C(R3)=C(R3), NH=C(R3), C(R3)=NH, NH=NH;
- B: C(R3), N(R2);
- D: C(R3), N(R2);
wobei mindestens eines der Glieder A, B oder D Stickstoff sein muss;
- n: unabhängig voneinander jeweils 1 oder 2;
- L: eine Binding, -C(=O)-, -C(=S)-, -C(=O)-N(R2)-, -C(=O)-O-, -S(O)₀₋₂-, -S(O)₀₋₂-N(R2)-, ein mono- oder bicyclisches Ringsystem, worin ein oder mehrere Ringglieder N(R3), O, S oder -C(=O)- sein können;
- M: -C(=O)-N(R2)-, -N(R2)-C(=O)-, -S(O)₀₋₂-, -S(O)₀₋₂-N(R2)-, -N(R2)-S(O)₀₋₂-, -C(=O)-O-, -O-C(=O)-;
und deren physiologisch verträgliche Salze.

Bevorzugt sind Verbindungen der Formel I, worin bedeuten
- R: Wasserstoff, (C₁-C₁₆)-Alkyl, (C₁-C₅)-Alkyloxy, (C₁-C₅)-Alkylthio, (C₁-C₅)- Alkylamino, Di-(C₂-C₈)-alkylamino, (C₀-C₄)-Alkylen-(C₆-C₁₀)-aryl, (C₀-C₄)- Alkylen-(C₅-C₁₂)-heteroaryl, (C₀-C₄)-Alkylen-(C₃-C₁₂)-heterocyclyl, (C₀-C₄)- Alkylen-(C₃-C₁₂)-cycloalkyl, ein bicyclisches (C₈-C₁₄)-Ringsystem, wobei Aryl, Heteroaryl, Heterocyclyl Cycloalkyl oder das bicyclisches (C₈-C₁₄)-Ringsystem, ein- oder mehrfach mit Halogen, (C₁-C₆)-Alkyl, (C₁-C₃)-Alkyloxy, Hydroxy, (C₁-C₆)-Alkylmercapto, Amino, (C₁-C₆)- Alkylamino, Di-(C₂-C₁₂)-alkylamino, Mono-(C₁-C₆)-alkylaminocarbonyl, Di-(C₂-C₈)-alkylarninocarbonyl, (C₁-C₆)-Alkoxycarbonyl, (C₁-C₆)- Alkylcarbonyl, Cyano, Trifluormethyl, Trifluormethyloxy, (C₁-C₆)- Alkylsulfonyl oder Aminosulfonyl substituiert sein können;
- R1: Wasserstoff, (C₁-C₁₀)-Alkyl, (C₁-C₁₀)-Alkyloxy, Amino, Mono-(C₁-C₁₀)- Alkylamino, Di-(C₂-C₁₂)-alkylamino, wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₅-C₁₂)- Heteroaryl, -(C₃-C₁₂)-Heterocyclyl oder -(C₃-C₁₂)-Cycloalkyl, wobei Aryl, Heteroaryl, Heterocyclyl oder Cycloalkyl gegebenenfalls ein- oder mehrfach durch Halogen, (C₁-C₆)-Alkyl, (C₁-C₃)₋Alkyloxy, Hydroxy, (C₁-C₆)-Alkylmercapto, Amino, (C₁- C₆)-Alkylamino, Di-(C₂-C₁₂)-alkylamino, substituiert sein können;
- R2: Wasserstoff, (C₁-C₁₆)-Alkyl, (C₀-C₄)-Alkylen-(C₆-C₁₀)-aryl;
- R3: Wasserstoff, (C₁-C₆)-Alkyl, (C₁-C₃)-Alkyloxy, Hydroxy, (C₁-C₆)-Alkylmercapto, Amino, (C₁-C₆)-Alkylamino, Di-(C₂-C₁₂)-alkylamino, Cyano, (C₁-C₆)- Alkylcarbonyl, Halogen, Trifluormethyl, Trifluormethyloxy, (C₁-C₆)- Alkylsulfonyl, Aminosulfonyl;
- A: O, S, N(R2), C(R3), C(R3)=C(R3);
- B: C(R3), N(R2);
- D: C(R3), N(R2);
wobei mindestens eines der Glieder A, B oder D Stickstoff sein muss;
- n: unabhängig voneinander jeweils 1 oder 2;
- L: eine Binding, -C(=O)-, -C(=S)-, -C(=O)-N(R2)-, -C(=O)-O-, -S(O)₀₋₂-, -S(O)₀₋₂-N(R2)-, ein mono- oder bicyclisches Ringsystem, worin ein oder mehrere Ringglieder N(R3), O, S oder -C(=O)- sein können;
- M: -C(=O)-N(R2)-, -N(R2)-C(=O)-, -S(O)₀₋₂-, -S(O)₀₋₂-N(R2)-, -N(R2)-S(O)₀₋₂, -C(=O)-O-, -O-C(=O)-;
und deren physiologisch verträgliche Salze.

Besonders bevorzugt sind Verbindungen der Formel I, mit n gleich 1, was die Verbindungen der Fomel Ia ergibt 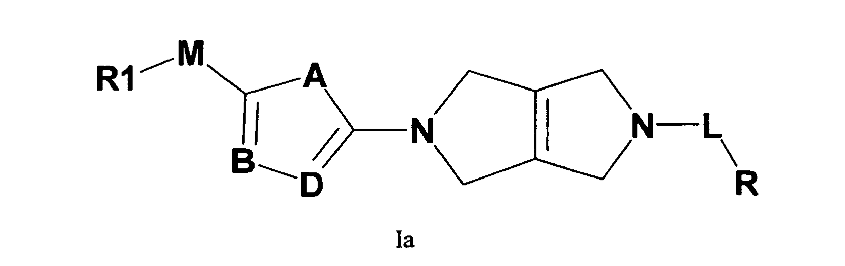 worin bedeuten
- R: Wasserstoff, (C₁-C₁₆)-Alkyl, (C₁-C₅)-Alkyloxy, (C₁-C₅)-Alkylthio, (C₁-C₅)- Alkylamino, Di-(C₂-C₈)-alkylamino, (C₀-C₄)-Alkylen-(C₆-C₁₀)-aryl, (C₀-C₄)- Alkylen-(C₅-C₁₂)-heteroaryl, (C₀-C₄)-Alkylen-(C₃-C₁₂)-heterocyclyl, (C₀-C₄)- Alkylen-(C₃-C₁₂)-cycloalkyl, ein bicyclisches (C₈-C₁₄)-Ringsystem, wobei Aryl, Heteroaryl, Heterocyclyl, Cycloalkyl oder das bicyclisches (C₈-C₁₄)-Ringsystem, ein- oder mehrfach mit Halogen, (C₁-C₆)-Alkyl, (C₁-C₃)-Alkyloxy, Hydroxy, (C₁-C₆)-Alkylmercapto, Amino, (C₁-C₆)- Alkylamino, Di-(C₂-C₁₂)-alkylamino, Mono-(C₁-C₆)₋alkylaminocarbonyl, Di-(C₂-C₈)-alkylaminocarbonyl, (C₁-C₆)-Alkoxycarbonyl, (C₁-C₆)- Alkylcarbonyl, Cyano, Trifluormethyl, Trifluormethyloxy, (C₁-C₆)- Alkylsulfonyl oder Aminosulfonyl substituiert sein können;
- R1: (C₁-C₁₀)-Alkyl, (C₁-C₁₀)-Alkyloxy, Amino, Mono-(C₁-C₁₀)-Alkylamino, Di-(C₂- C₁₂)-alkylamino, wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₅-C₁₂)- Heteroaryl, -(C₃-C₁₂)-Heterocyclyl oder -(C₃-C₁₂)-Cycloalkyl, wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
- R2: Wasserstoff, (C₁-C₁₆)-Alkyl, (C₀-C₄)-Alkylen-(C₆-C₁₀)-aryl;
- R3: Wasserstoff, (C₁-C₆)-Alkyl, (C₁-C₃)-Alkyloxy, Hydroxy, (C₁-C₆)-Alkylmercapto, Amino, (C₁-C₆)-Alkylamino, Di-(C₂-C₁₂)-alkylarnino, Cyano, (C₁-C₆)- Alkylcarbonyl, Halogen, Trifluormethyl, Trifluormethyloxy, (C₁-C₆)- Alkylsulfonyl, Aminosulfonyl;
- A: O, S, N(R2), C(R3), C(R3)=C(R3);
- B: C(R3), N(R2);
- D: C(R3), N(R2);
wobei mindestens eines der Glieder A, B oder D Stickstoff sein muss;
- L: eine Binding, -C(=O)-, -C(=S)-, -C(=O)-N(R2)-, -C(=O)-O-, -S(O)₀₋₂-, -S(O)₀₋₂-N(R2)-, ein mono- oder bicyclisches Ringsystem, worin ein oder mehrere Ringglieder N(R3), O, S oder -C(=O)- sein können;
- M: -C(=O)-N(R2)-, N(R2)-C(=O)-, -S(O)₀₋₂-, -S(O)₀₋₂-N(R2)-, -N(R2)-S(O)₀₋₂-, -O-C(=O)-;
und deren physiologisch verträgliche Salze.

Ganz besonders bevorzugt sind Verbindungen der Formel Ia, 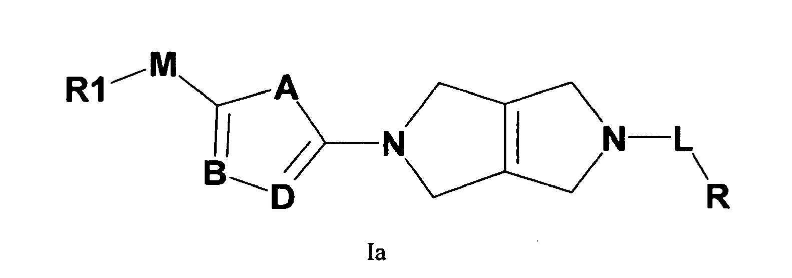 worin bedeuten
- R: Wasserstoff, (C₁-C₁₆)-Alkyl, (C₁-C₅)-Alkyloxy, (C₁-C₅)-Alkylthio, (C₁-C₅)- Alkylamino, Di-(C₂-C₈)-alkylamino, (C₀-C₄)-Alkylen-(C₆-C₁₀)-aryl, (C₀-C₄)- Alkylen-(C₅-C₁₂)-heteroaryl, (C₀-C₄)-Alkylen-(C₃-C₁₂)-heterocyclyl, (C₀-C₄)- Alkylen-(C₃-C₁₂)-cycloalkyl, ein bicyclisches (C₈-C₁₄)-Ringsystem, wobei Aryl, Heteroaryl, Heterocyclyl, Cycloalkyl oder das bicyclisches (C₈-C₁₄)-Ringsystem, ein- oder mehrfach mit Halogen, (C₁-C₆)-Alkyl, (C₁-C₃)-Alkyloxy, Hydroxy, (C₁-C₆)-Alkylmercapto, Amino, (C₁-C₆)- Alkylamino, Di-(C₂-C₁₂)-alkylamino, Mono-(C₁-C₆)-alkylaminocarbonyl, Di-(C₂-C₈)-alkylaminocarbonyl, (C₁-C₆)-Alkoxycarbonyl, (C₁-C₆)- Alkylcarbonyl, Cyano, Trifluormethyl, Trifluormethyloxy, (C₁-C₆)- Alkylsulfonyl oder Aminosulfonyl substituiert sein können;
- R1: (C₁-C₁₀)-Alkyl, (C₁-C₁₀)-Alkyloxy, Amino, Mono-(C₁-C₁₀)-Alkylamino, Di-(C₂- C₁₂)-alkylamino, wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₅-C₁₂)- Heteroaryl, -(C₃-C₁₂)-Heterocyclyl oder -(C₃-C₁₂)-Cycloalkyl, wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
- R2: Wasserstoff, (C₁-C₁₆)-Alkyl, (C₀-C₄)-Alkylen-(C₆-C₁₀)-aryl;
- R3: Wasserstoff, (C₁-C₆)-Alkyl, (C₁-C₃)-Alkyloxy, Hydroxy, (C₁-C₆)-Alkylmercapto, Amino, (C₁-C₆)-Alkylamino, Di-(C₂-C₁₂)-alkylamino, Cyano, (C₁-C₆)- Alkylcarbonyl, Halogen, Trifluormethyl, Trifluormethyloxy, (C₁-C₆)- Alkylsulfonyl, Aminosulfonyl;
- A: S, C(R3)=C(R3);
- B: C(R3);
- D: N(R2);
- L: eine Binding, -C(=O)-, -C(=S)-, -C(=O)-N(R2)-, -C(=O)-O-, -S(O)₀₋₂-, -S(O)₀₋₂-N(R2)-, ein mono- oder bicyclisches Ringsystem, worin ein oder mehrere Ringglieder N(R3), O, S oder -C(=O)- sein können;
- M: -C(=O)-N(R2)-, N(R2)-C(=O)-, -S(O)₀₋₂-, -S(O)₀₋₂-N(R2)-, -N(R2)-S(O)₀₋₂-, -O-C(=O)-;
und deren physiologisch verträgliche Salze.

Weiter ganz besonders bevorzugt sind Verbindungen der Formel Ia, 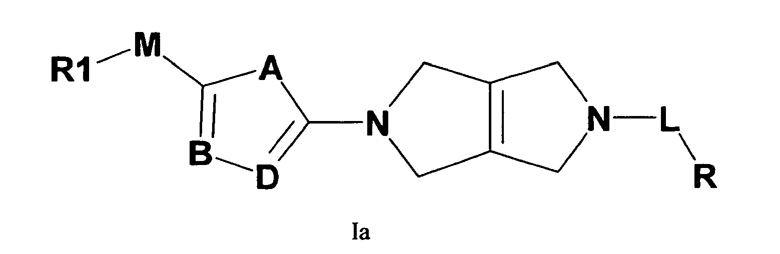 worin bedeuten
- R: (C₁-C₁₆)-Alkyl, (C₁-C₅)-Alkyloxy, (C₀-C₄)-Alkylen-(C₆-C₁₀)-aryl, ein bicycli- sches (C₈-C₁₄)-Ringsystem, wobei aryl oder das bicyclisches (C₈-C₁₄)-Ringsystem ein- oder mehrfach mit Halogen, (C₁-C₆)-Alkyl, (C₁-C₃)-Alkyloxy, Hydroxy, (C₁- C₆)-Alkylmercapto, Amino, (C₁-C₆)-Alkylamino, Di-(C₂-C₁₂)- alkylamino, Mono-(C₁-C₆)-alkylaminocarbonyl, Di-(C₂-C₈)- alkylaminocarbonyl, (C₁-C₆)-Alkoxycarbonyl, (C₁-C₆)-Alkylcarbonyl, Cyano, Trifluormethyl, Trifluormethyloxy, (C₁-C₆)-Alkylsulfonyl oder Aminosulfonyl substituiert sein können;
- R1: (C₁-C₁₀)-Alkyl, (C₁-C₁₀)-Alkyloxy, Amino, Mono-(C₁-C₁₀)-Alkylamino, Di-(C₂- C₁₂)-alkylamino, wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₅-C₁₂)- Heteroaryl, -(C₃-C₁₂)-Heteroeyclyl oder -(C₃-C₁₂)-Cycloalkyl, wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
- R2: Wasserstoff, (C₁-C₁₆)-Alkyl, (C₀-C₄)-Alkylen-(C₆-C₁₀)-aryl;
- R3: Wasserstoff, (C₁-C₆)-Alkyl, (C₁-C₃)-Alkyloxy, Hydroxy, (C₁-C₆)-Alkylmercapto, Amino, (C₁-C₆)-Alkylamino, Di-(C₂-C₁₂)-alkylamino, Cyano, (C₁-C₆)- Alkylcarbonyl, Halogen, Trifluormethyl, Trifluormethyloxy, (C₁-C₆)- Alkylsulfonyl, Aminosulfonyl;
- A: S, C(R3)=C(R3);
- B: C(R3);
- D: N(R2);
- L: eine Binding, -C(=O)-;
- M: -C(=O)-N(R2)-, -O-C(=O)-;
und deren physiologisch verträgliche Salze.

In einer Ausführungsform sind Verbindungen der Formel I bevorzugt, in denen n jeweils gleich 1 ist.

In einer Ausführungsform sind Verbindungen der Formel I bevorzugt, in denen L gleich C=O ist.

In einer Ausführungsform sind Verbindungen der Formel I bevorzugt, in denen M gleich - C(=O)-N(R2) ist.

In einer Ausführungsform sind Verbindungen der Formel I bevorzugt, in denen bedeuten: A gleich S, D gleich N und B gleich CH.

In einer Ausführungsform sind Verbindungen der Formel I bevorzugt, in denen bedeuten: A gleich C(R3)=C(R3), D gleich N und B gleich CH.

In einer Ausführungsform sind Verbindungen der Formel I bevorzugt, in denen bedeuten: A gleich CH=CH, D gleich N und B gleich CH.

In einer Ausführungsform sind Verbindungen der Formel I bevorzugt, in denen bedeuten: A gleich C(R3)=C(R3), D gleich N und B gleich N.

In einer Ausführungsform sind Verbindungen der Formel I bevorzugt, in denen bedeuten: A gleich CH=CH, D gleich N und B gleich N.

Die Erfindung betrifft Verbindungen der Formel I in Form ihrer Salze, Racemate, racemischen Mischungen und reinen Enantiomere, und deren Diastereomere und Mischungen davon.

Die Alkylreste in den Substituenten R, R1, R2, und R3 können entweder geradkettig oder verzweigt sein.

Halogen steht für. Fluor, Chlor, Brom oder Iod, insbesondere Fluor oder Chlor.

Aryl steht für einen monocyclischen oder bicyclischen aromatischen Kohlenwasserstoffrest mit 6 bis 10 Ringatomen, der unabhängig durch einen bis vier, vorzugsweise einen oder zwei, beschriebene Substituenten substituiert sein kann.

Heteroaryl steht für einen monocyclischen oder bicyclischen Rest mit 5 bis 12 Ringatomen mit wenigstens einem aromatischen Ring mit einem, zwei oder drei Ringheteroatomen ausgewählt aus N, O und S, wobei die restlichen Ringatome C sind.

Cycloalkyl steht für ein gesättigtes oder teilweise ungesättigtes Ringsystem (das ausschließlich Kohlenstoffatome enthält), das ein oder mehrere Ringe umfaßt.

Heterocyclyl steht für ein gesättigtes oder teilweise ungesättigtes Ringsystem (das wenigstens ein Heteroatom enthält), das ein oder mehrere Ringe umfaßt.

Bicyclyl steht für ein bicyclisches gesättigtes oder teilweise ungesättigtes Ringsystem, wobei die einzelnen Glieder der Ringsysteme ausschließlich Kohlenstoffatome oder ein, zwei oder drei Ringheteroatome ausgewählt aus N, O und S enthalten können, wobei die restlichen Ringatome C sind. Bei einem der Ringe in dem bicyclischen System kann es sich auch um einen kondensierten aromatischen Ring wie Benzol handeln

Können Reste oder Substituenten mehrfach in den Verbindungen der Formel I auftreten (wie zum Beispiel "n"), so können sie alle unabhängig voneinander die angegebenen Bedeutungen haben und gleich oder verschieden sein.

Physiologisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein physiologisch verträgliches Anion oder Kation aufweisen. Geeignete physiologisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Apfel-, Methansulfon-, Bernstein-, p-Toluolsulfon-, und Weinsäure. Für medizinische Zwecke wird in besonders bevorzugter Weise das Chlorsalz verwendet. Geeignete physiologisch verträgliche basische Salze sind Ammoniumsalze, Alkalimetallsalze (wie Natrium- und Kaliumsalze), Erdalkalisalze (wie Magnesium- und Calciumsalze), Zinksalze, Trometarnol (2-Amino-2-hydroxymethyl-1,3-propandiol)-, Diethanolamin-, Lysin-, Arginin-, Cholin-, Meglumin- oder Ethylendiamin-Salze.

Salze mit einem nicht physiologisch verträglichen Anion oder Kation gehören ebenfalls in den Rahmen der Erfindung als nützliche Zwischenprodukte für die Herstellung oder Reinigung physiologisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze und wie hierin beschrieben.

Die Verbindungen der Formel I und deren physiologisch verträgliche Salze stellen ideale Arzneimittel zur Behandlung von erhöhten Lipidkonzentrationen im Blut und in Geweben, dem metabolischen Syndrom, Obesitas, Diabetes, Insulinresistenz, der Dysregulation von LDL, HLD und VLDL oder Herzkreislauferkrankungen Lipidstoffwechselstörungen, insbesondere von Hyperlipidämie dar.

Die Verbindung(en) der Formel I können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,1 mg bis 100 mg (typischerweise von 0,1 mg bis 50 mg) pro Tag pro Kilogramm Körpergewicht, z.B. 0,1-10 mg/kg/Tag. Tabletten oder Kapseln, können beispielsweise von 0,01 bis 100 mg, typischerweise von 0,02 bis 50 mg enthalten. Zur Prophylaxe oder Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive

Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

### Kombinationen mit anderen Medikamenten

Die erfindungsgemäßen Verbindungen können alleine oder in Kombination mit einem oder mehreren weiteren pharmakologischen Wirkstoffen, die beispielsweise günstige Wirkungen auf Stoffwechselstörungen bzw. häufig damit assoziierten Erkrankungen haven, verabreicht werden. Beispiele für solche Medikamente sind
1. blutzuckersenkende Medikamente, Antidiabetika,
2. Wirkstoffe zur Behandlung von Dyslipidämien,
3. antiatherosklerotische Medikamente,
4. Mittel gegen Obesitas,
5. entzündungshemmende Wirkstoffe
6. Wirkstoffe zur Behandlung von malignen Tumoren
7. antithrombotische Wirkstoffe
8. Wirkstoffe zur Behandlung von Bluthochdruck
9. Wirkstoffe zur Behandlung von Herzinsuffizienz und
10. Wirkstoffe zur Behandlung und/oder Prävention von durch Diabetes verursachten bzw. mit Diabetes assoziierten Komplikationen.

Sie lassen sich mit den erfindungsgemäßen Verbindungen der Formel I insbesondere zur synergistischen Wirkungsverbesserung kombinieren. Die Verabreichung der Wirkstoffkombination kann entweder durch getrennte Verabreichung der Wirkstoffe an den Patienten oder in Form von Kombinationsprodukten, bei denen mehrere Wirkstoffe in einer pharmazeutischen Zubereitung vorhanden sind, erfolgen.

Als weitere Wirkstoffe für die Kombinationspräparate sind insbesonders geeignet:
Alle Antidiabetika, die in der Roten Liste 2006, Kapitel 12 genannt sind; alle Abmagerungsmittel/Appetitzügler, die in der Roten Liste 2005, Kapitel 1 gennant sind; alle Lipidsenker, die in der Roten Liste 2006, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964 oder solche, wie sie in WO2005005477 (Novo Nordisk) beschrieben sind, schnell wirkende Insuline (siehe US 6,221,633), inhalierbare Insuline, wie z. B. Exubera ^{®} oder orale Insuline, wie z. B. IN-105 (Nobex) oder Oral-lyn ™ (Generex Biotechnology), GLP-1-Derivate wie z.B. Exenatide, Liraglutide oder diejenigen die in WO 98/08871, WO2005027978 von Novo Nordisk A/S, in WO 01/04156 von Zealand oder in WO 00/34331 von Beaufour-Ipsen offenbart wurden, Pramlintide Acetat (Symlin; Amylin Pharmaceuticals), sowie oral wirksame hypoglykämische Wirkstoffe.

Die oral wirksamen hypoglykämischen Wirkstoffe umfassen vorzugsweise Sulfonylharnstoffe, Biguanidine,
Meglitinide,
Oxadiazolidindione,
Thiazolidindione,
Glukosidase-Inhibitoren,
Hemmstoffe der Glykogenphosphorylase,
Glukagon-Antagonisten,
Glukokinaseaktivatoren,
Inhibitoren der Fructose-1,6-bisphosphatase
Modulatoren des Glukosetransporters-4 (GLUT4),
Inhibitoren der Glutamin-Fructose-6-Phosphat-Amidotransferase (GFAT),
GLP-1-Agonisten, Kaliumkanalöffner, wie z.B. diejenigen, die in WO 97/26265 und WO 99/03861 von Novo Nordisk A/S offenbart wurden,
Inhibitoren der Dipeptidylpeptidase-IV (DPP-IV),
Insulin-Sensitizer,
Inhibitoren von Leberenzymen, die an der Stimulation der Glukoneogenese und/oder
Glykogenolyse beteiligt sind,
Modulatoren der Glukoseaufnahme, des Glukosetransports und der Glukoserückresorption,
Hemmstoffe der 11ß-HSD1,
Inhibitoren der Protein-Tyrosin-Phosphatase-1B (PTP 1 B),
Modulatoren des natrium-abhängigen Glukosetransporters 1 oder 2 (SGLT1, SGLT2), den Fettstoffwechsel verändernde Verbindungen wie antihyperlipidämische Wirkstoffe und antilipidämische Wirkstoffe,
Verbindungen, die die Nahrungsmitteleinnahme verringern,
Verbindungen, die die Thermogenese erhöhen,
PPAR- und RXR-Modulatoren und
Wirkstoffe, die auf den ATP-abhängigen Kaliumkanal der Betazellen wirken.

Bei einer Ausführungsform der Erfindung wird die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin, L-659699
verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692), MD-0727 (Microbia Inc., WO2005021497) oder mit Verbindungen, wie in WO2002066464 (Kotobuki Pharmaceutical Co. Ltd.) oder WO2005062824 (Merck & Co.) oder WO2005061451 und WO2005061452 (AstraZeneca AB) beschrieben, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, G1 262570, R-483, CS-011 (Rivoglitazon) verabreicht.

Bei einer Ausführungsform der Erfindung wird die Verbindung der Formel I in Kombination mit PPAR alpha Agonist, wie z.B. GW9578, GW-590735, K-111, LY-674, KRP-101, DRF-10945 verabreicht.

Bei einer Ausführungsform der Erfindung wird die Verbindung der Formel I in Kombination mit einem gemischten PPAR alpha/gamma Agonisten, wie z.B. Muraglitazar, Tesaglitazar, Naveglitazar, LY-510929, ONO-5129, E-3030, AVE 8042, AVE 8134, AVE 0847, oder wie in PCT/US 00/11833, PCT/US 00/11490, DE10142734.4 oder in J.P.Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516 verabreicht.

Bei einer Ausführungsform wird die Verbindung der Formel I in Kombination mit Metaglidasen oder mit MBX-2044 oder anderen partiellen PPAR gamma Agonisten/Antagonisten verabreicht

Bei einer Ausführungsform der Erfindung wird die Verbindung der Formel I in Kombination mit einem Fibrat, wie z.B. Fenofibrat, Clofibrat, Bezafibrat, verabreicht.

Bei einer Ausführungsform der Erfindung wird die Verbindung der Formel I in Kombination mit einem MTP-Inhibitor, wie z.B. Implitapide , BMS-201038, R-103757 oder solchen wie in WO2005085226 beschrieben, verabreicht.

Bei einer Ausführungsform der Erfindung wird die Verbindung der Formel I in Kombination mit einem CETP-Inhibitor, wie z.B. Torcetrapib oder JTT-705 , verabreicht.

Bei einer Ausführungsform der Erfindung wird die Verbindung der Formel I in Kombination mit Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9 beschrieben, verabreicht.

Bei einer Ausführungsform der Erfindung wird die Verbindung der Formel I in Kombination mit einem polymeren Gallensäureadsorber, wie z.B. Cholestyramin, Colesevelam, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586, oder solchen wie in WO2005097738 beschrieben, verabreicht.

Bei einer Ausführungsform wird die Verbindung der Formel I in Kombination mit Omacor® (Omega-3-Fettsäuren; hochkonzentrierte Ethylester der Eicosapentaensäure und der Docosahexaensäure) verabreicht.

Bei einer Ausführungsform der Erfindung wird die Verbindung der Formel I in Kombination mit einem ACAT-Inhibitor, wie z.B. Avasimibe, verabreicht.

Bei einer Ausführungsform der Erfindung wird die Verbindung der Formel I in Kombination mit einem Antioxidans, wie z.B. OPC-14117, Probucol, Tocopherol, Ascorbinsäure, ß-Caroten oder Selen verabreicht.

Bei einer Ausführungsform der Erfindung wird die Verbindung der Formel I in Kombination mit einem Vitamin, wie z. B. Vitamin B6 oder Vitamin B12 verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform der Erfindung wird die Verbindung der Formel I in Kombination mit einem Squalen Synthetase Inhibitor, wie z.B. BMS-188494 oder wie in WO2005077907 beschrieben, verabreicht.

Bei einer Ausführungsform der Erfindung wird die Verbindung der Formel I in Kombination mit einem Lipoprotein(a) antagonist, wie z.B. Gemcabene (CI-1027) verabreicht.

Bei einer Ausführungsform der Erfindung wird die Verbindung der Formel I in Kombination mit einem HM74A Rezeptor Agonisten, wie z.B. Nicotinsäure, verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit Insulin verabreicht.

Bei einer Ausführungsform wird die Verbindung der Formel I in Kombination mit einem Sulfonylharnstoff, wie z.B. Tolbutamid, Glibenclamid, Glipizid oder Glimepirid verabreicht.

Bei einer Ausführungsform wird die Verbindung der Formel I in Kombination mit einem Biguanid, wie z.B. Metformin, verabreicht.

Bei wieder einer Ausführungsform wird die Verbindung der Formel I in Kombination mit einem Meglitinid, wie z.B. Repaglinide oder Nateglinid, verabreicht.

Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform wird die Verbindung der Formel I in Kombination mit einem α-Glukosidase-Inhibitor, wie z.B. Miglitol oder Acarbose, verabreicht.

Bei einer Ausführungsform wird die Verbindung der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsforin wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer Ausführungsform wird die Verbindung der Formel I in Kombination mit einem Hemmstoff der Glykogenphosphorylase, wie z.B. PSN-357 oder FR-258900 oder solchen wie in WO2003084922, WO2004007455, WO2005073229-31, WO2005067932 beschrieben, verabreicht.

Bei einer Ausführungsform wird die Verbindung der Formel I in Kombination mit Glukagon-Rezeptor-Antagonisten, wie z.B. A-770077 oder NNC-25-2504 oder wie in WO2004100875, WO2005065680, beschrieben, verabreicht.

Bei einer Ausführungsform wird die Verbindung der Formel I in Kombination mit Aktivatoren der Glukokinase, wie z. B. LY-2121260 (WO2004063179), PSN-105, PSN-110, GKA-50 oder solchen wie sie z. B. in WO2004072031 oder WO2004072066 oder WO2005080360 beschrieben sind, verabreicht.

Bei einer Ausführungsform wird die Verbindung der Formel I in Kombination mit einem Inhibitor der Glukoneogenese, wie z. B. FR-225654, verabreicht.

Bei einer Ausführungsform wird die Verbindung der Formel I in Kombination mit Inhibitoren der Fructose-1,6-bisphosphatase (FBPase) wie z.B. CS-917, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Saxagliptin ((BMS-477118), GSK-823093, PSN-9301, SYR-322, SYR-619, TA-6666, TS-021, GRC-8200, GW-825964X oder wie sie in WO2003074500, WO2003106456, WO200450658, WO2005058901, WO2005012312, WO2005/012308, PCT/EP2005/007821, PCT/EP2005/008005, PCT/EP2005/008002, PCT/EP2005/008004, PCT/EP2005/008283, DE 10 2005 012874.2 oder DE 10 2005 012873.4 beschrieben sind, verabreicht.

Bei einer Ausführungsform wird die Verbindung der Formel I in Kombination mit Hemmstoffen der 11-beta-Hydroxysteroid-Dehydrogenase-1 (11β-HSD1), wie z. B. BVT-2733 oder solche, wie sie z. B. in WO200190090-94, WO200343999, WO2004112782, WO200344000, WO200344009, WO2004112779 WO2004113310, WO2004103980, WO2004112784, WO2003065983, WO2003104207, WO2003104208, WO2004106294, WO2004011410, WO2004033427, WO2004041264, WO2004037251, WO2004056744, WO2004065351, WO2004089367, WO2004089380, WO2004089470-71, WO2004089896, WO2005016877, WO2005097759 beschrieben sind, verabreicht.

Bei einer Ausführungsform wird die Verbindung der Formel I in Kombination mit Inhibitoren der Protein-Tyrosin-Phosphatase-1B (PTP1B), wie sie z. B. in WO200119830-31, WO200117516, WO2004506446, WO2005012295, WO2005116003, WO2005116003, WO2006007959, DE 10 2004 060542.4, WO2007009911, WO2007028145, WO2007067612-615, WO2007081755, WO2007115058 beschrieben sind, verabreicht.

Bei einer Ausführungsform wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268 und SAR 7226 oder wie sie z. B. in WO2004007517, WO200452903, WO200452902, PCT/EP2005/005959, WO2005085237, JP2004359630 oder von A. L. Handlon in Expert Opin. Ther. Patents (2005) 15(11), 1531-1540 beschrieben sind, verabreicht.

Bei einer Ausführungsform wird die Verbindung der Formel I in Kombination mit Modulatoren des GPR40 verabreicht.

Bei einer Ausführungsform wird die Verbindung der Formel I in Kombination mit Inhibitoren der hormon-sensitiven Lipase (HSL), wie z. B. in WO2005073199 beschrieben, verabreicht.

Bei einer Ausführungsform wird die Verbindung der Formel I in Kombination mit Hemmstoffen der Acetyl-CoA Carboxylase (ACC) wie z. B. solchen wie in WO199946262, WO200372197, WO2003072197, WO2005044814 beschrieben, verabreicht.

Bei einer Ausführungsform wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform wird die Verbindung der Formel I in Kombination mit einem Inhibitor der Glykogen Synthase Kinase-3 beta (GSK-3 beta), wie z. B. in US2005222220, WO2005085230, PCT/EP2005/005346, WO2003078403, WO2004022544, WO2003106410, WO2005058908, US2005038023, WO2005009997, US2005026984, WO2005000836, WO2004106343, EP1460075, WO2004014910, WO2003076442, WO2005087727, WO2004046117 beschrieben.

Bei einer Ausführungsform wird die Verbindung der Formel I in Kombination mit einem Inhibitor der Protein Kinase C beta (PKC beta), wie z. B. Ruboxistaurin, verabreicht.

Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Inhibitoren der "I-kappaB kinase" (IKK Inhibitoren), wie sie z. B. in WO2001000610, WO2001030774, WO2004022553, WO2005097129 beschrieben sind, verabreicht.

Bei einer Ausführungsform wird die Verbindung der Formel I in Kombination mit Modulatoren des Glucocorticoidrezeptors, wie sie z. B. in WO2005090336 beschrieben sind, verabreicht.

Bei einer weiteren Ausführungsform wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolie Research (2001), 33(9), 554-558);
NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
Peptid YY 3-36 (PYY3-36) oder analoge Verbindungen wie z. B. CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in WO2005080424 beschrieben sind;
Cannabinoid Rezeptor 1 Antagonisten (wie z.B. Rimonabant, SR147778 oder solche wie sie in z. B. EP 0656354, WO 00/15609, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509, WO2005077897 beschrieben sind);
MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)) oder LB53280, LB53279, LB53278 oder THIQ, MB243, RY764, CHIR-785, PT-141 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, EP1538159, WO2004072076, WO2004072077 beschrieben sind;
Orexin-Rezeptor Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403, WO2005075458 beschrieben sind);
Histamin H3 Rezeptor Agonisten (z. B. 3-Cyclohexyl-1-(4,4-dimethyl-1,4,6,7-tetrahydroimidazo[4,5-c]pyridin-5-yl)-propan-1-on Oxalsäuresalz (WO 00/63208) oder solche, wie sie in WO200064884, WO2005082893 beschrieben sind);
CRF-Antagonisten (z.B. [2-Methyl-9-(2,4,6-trimethyl-phenyl)-9H-1,3,9-triaza-fluoren-4-yl]-dipropyl-amin (WO 00/66585));
CRF BP-Antagonisten (z.B. Urocortin);
Urocortin-Agonisten;
β3-Agonisten (wie z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451));
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2003/15769, WO2005085200, WO2005019240, WO2004011438, WO2004012648, WO2003015769, WO2004072025, WO2005070898, WO2005070925, WO2004039780, WO2003033476, WO2002006245, WO2002002744, WO2003004027, FR2868780 beschrieben sind);
CCK-A Agonisten (wie z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525) oder SR-146131 (WO 0244150) oder SSR-125180);
Serotonin-Wiederaufnahme-Inhibitoren (z.B. Dexfenfluramine);
gemischte Sertonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
5-HT2C Rezeptor Agonisten (wie z.B. APD-356 oder BVT-933 oder solche, wie sie in WO200077010, WO20077001-02, WO2005019180, WO2003064423, WO200242304, WO2005082859 beschrieben sind);
5-HT6 Rezeptor Antagonisten, wie sie z.B. in WO2005058858 beschrieben sind; Bombesin-Rezeptor Agonisten (BRS-3 Agonisten;
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695)); Growth Hormone Secretagogue Receptor Antagonisten (Ghrelin Antagonisten) wie z. B. A-778193 oder solchen, wie sie in WO2005030734 beschrieben sind;
TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Bromocriptin, Doprexin);
Lipase/Amylase-Inhibitoren (z.B. WO 00/40569);
Inhibitoren der Diacylglycerol O-Acyltransferasen (DGATs) wie z. B. in US2004/0224997, WO2004094618, WO200058491, WO2005044250, WO2005072740, JP2005206492, WO2005013907 beschrieben;
Inhibitoren der Fettsäuresynthase (FAS) wie z.B. C75 oder solchen, wie in WO2004005277 beschrieben;
Oxyntomodulin;
Oleoyl-Estron
oder Agonisten des Schilddrüsenhormonrezeptors (thyroid hormone receptor agonists) wie z. B: KB-2115 oder solche, wie in WO20058279, WO200172692, WO200194293, WO2003084915, WO2004018421, WO2005092316 beschrieben, verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Mazindol oder Phentermin.

Bei einer Ausführungsform wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

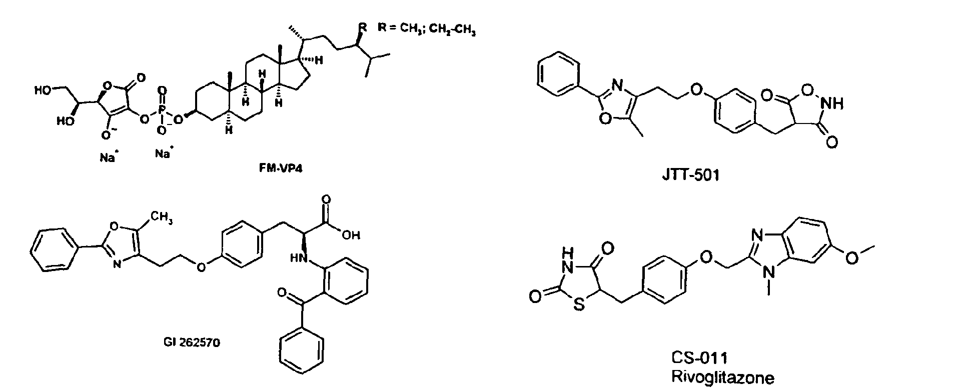

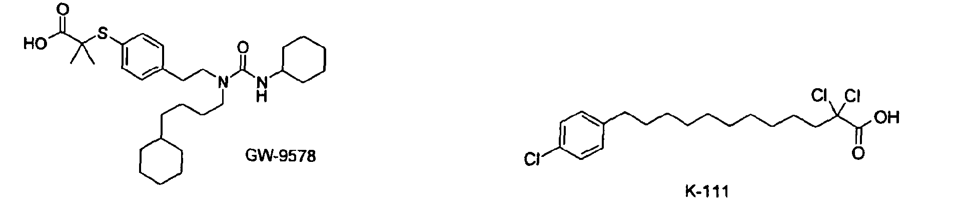

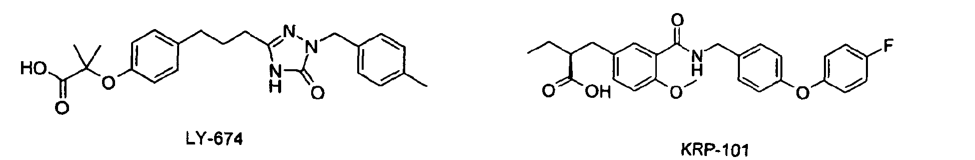

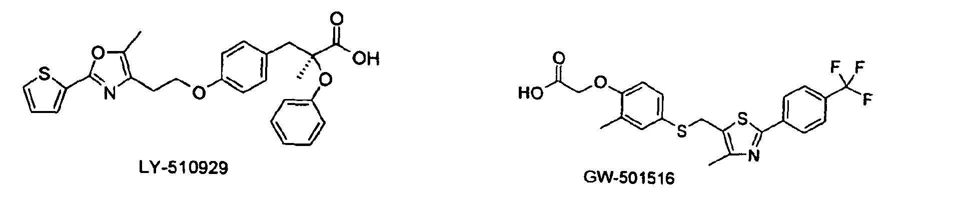

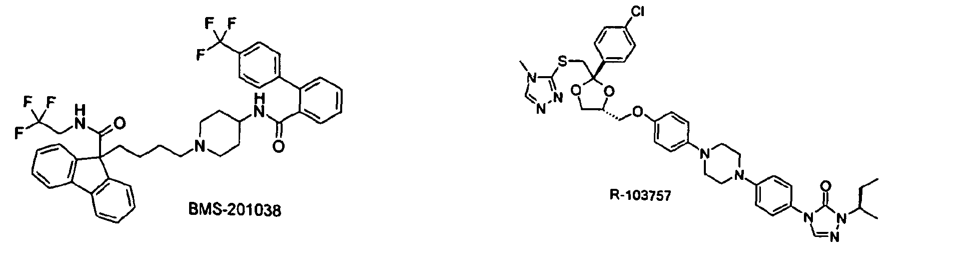

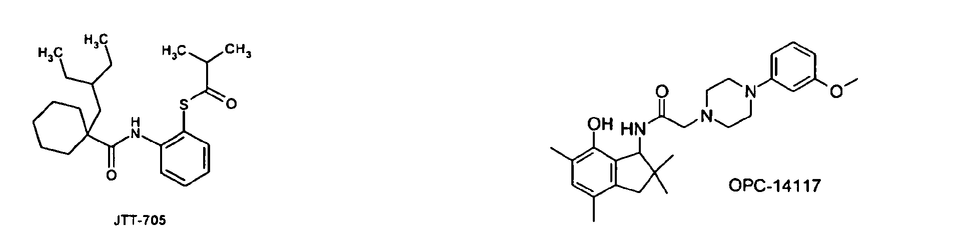

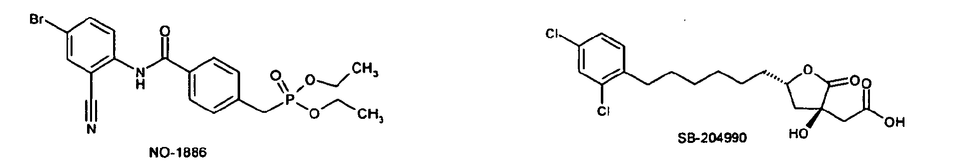

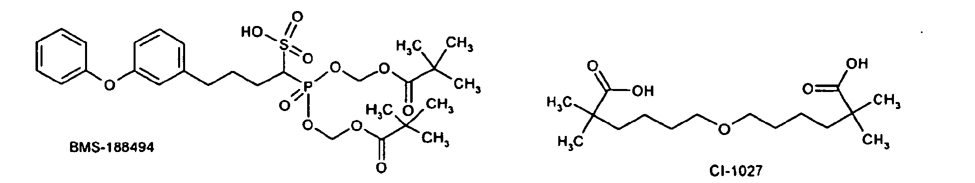

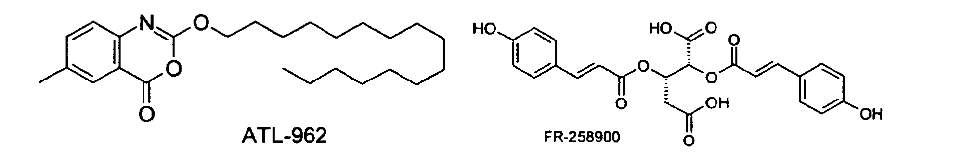

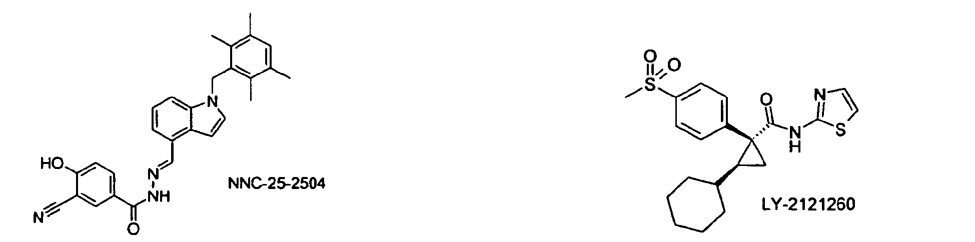

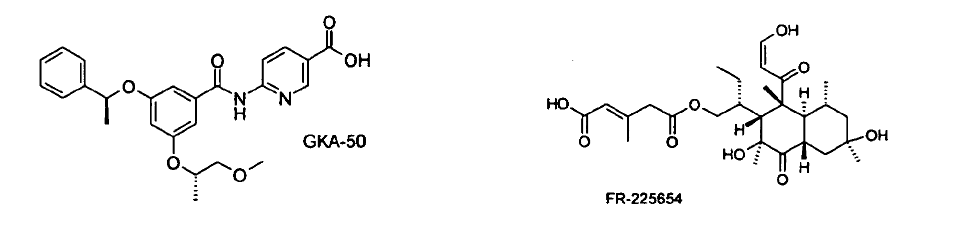

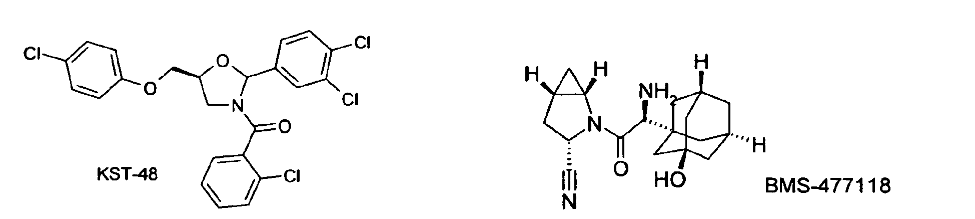

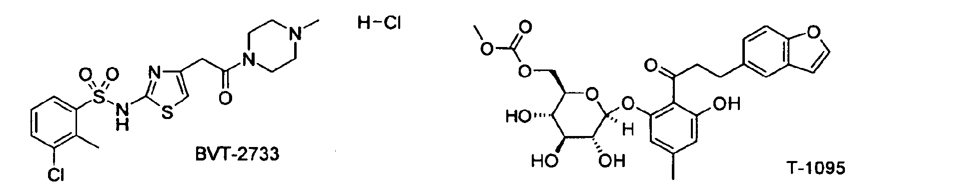

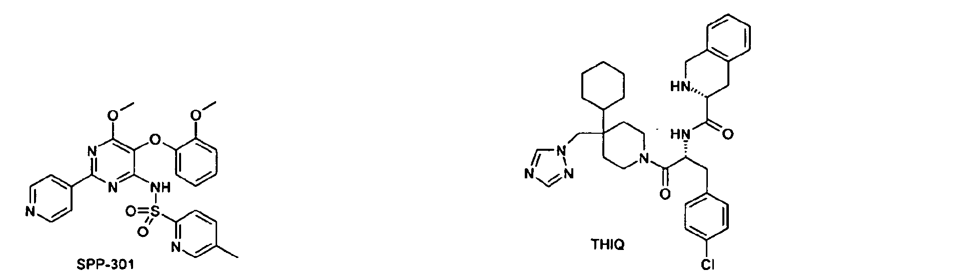

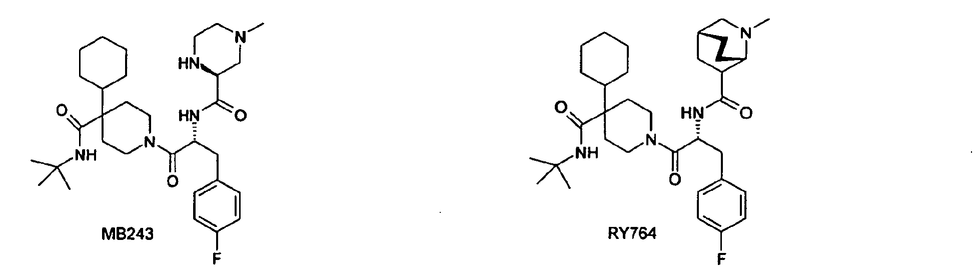

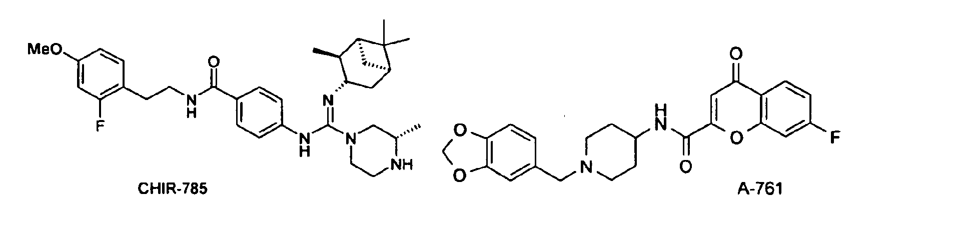

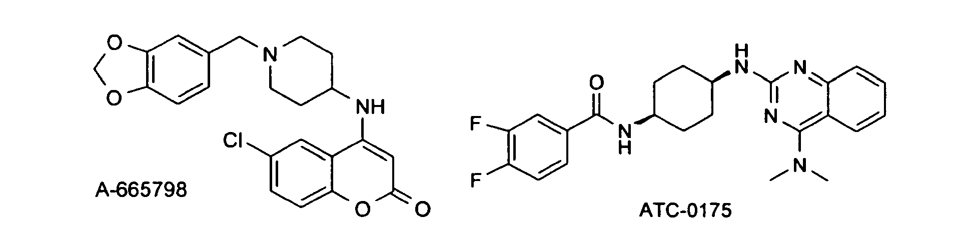

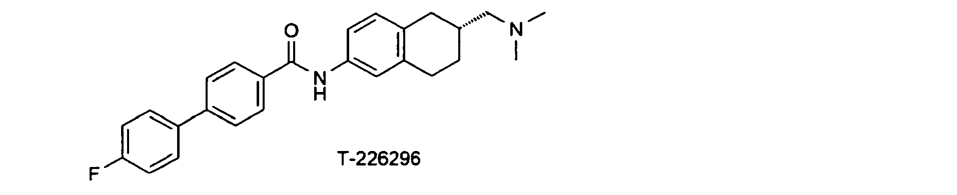

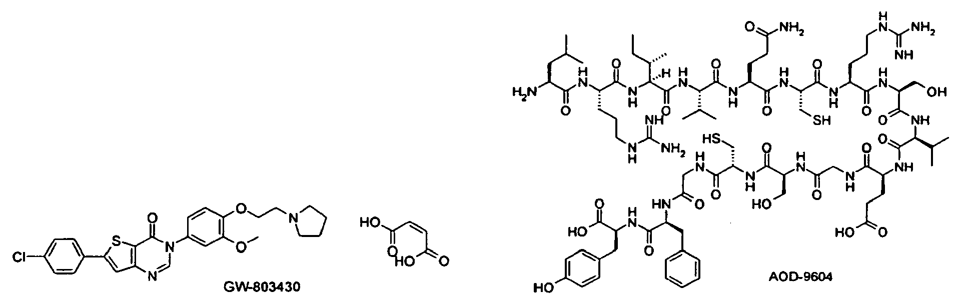

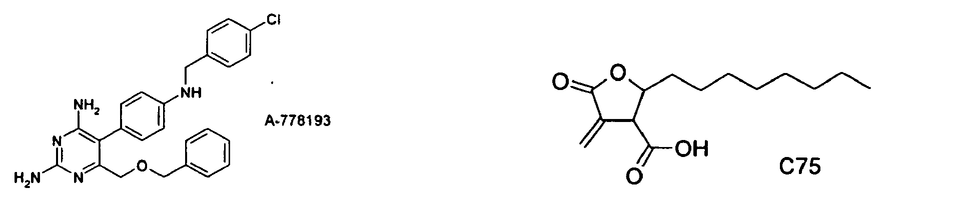

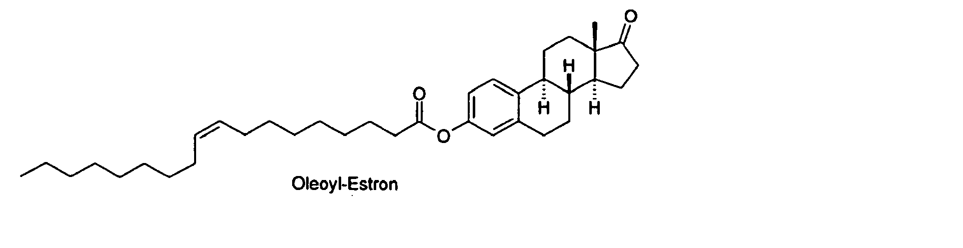

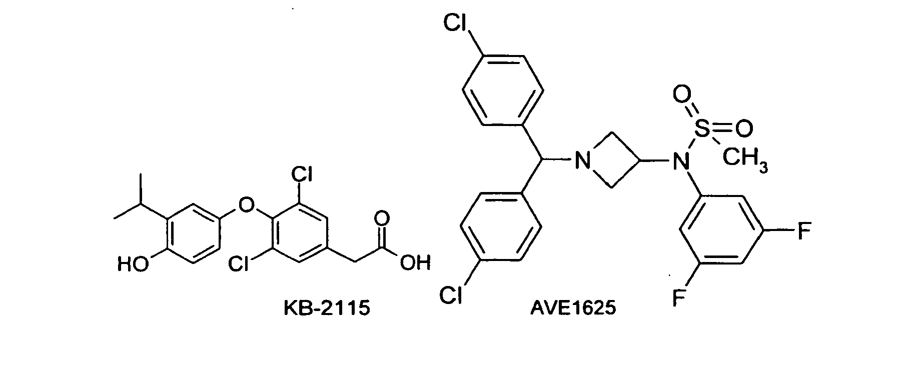

Gegenstand der Erfindung sind weiterhin sowohl Stereoisomerengemische der Formel I, als auch die reinen Stereoisomere der Formel I, sowie Diastereomerengemische der Formel I als auch die reinen Diastereomere. Die Trennung der Gemische erfolgt auf chromatographischem Weg.

Die nachfolgenden Beispiele dienen zur näheren Erläuterung der Erfindung, ohne dieselbe auf in den Beispielen beschriebene Produkte und Ausführungsformen einzuschränken.

Die Wirksamkeit der erfindungsgemäßen Verbindungen der Formel I wurden in dem folgenden Assaysystem mit mikrosomalen Enzymen untersucht:
Die Lebern von männlichen Wistar-Ratten, die (zur Induktion der Expression von SCD1) mit einer kohlenhydratreichen, fettarmen Nahrung gefüttert worden waren, wurden mit einem Potter-Gewebehomogenisator und Differentialzentrifugation in einem Puffer homogenisiert, der 250 mM/l Saccharose und 5 mM/l HEPES (pH 7,0) enthielt. Die resuspendierte Mikrosomenfraktion wurde bei -80°C aufbewahrt. Die Stearoyl-CoA-Desaturase-Aktivität wurde in einem Dünnschichtchromatographieassay mit 1-¹⁴C-markierter Stearinsäure bestimmt. Kurz gesagt wurden die jeweiligen erfindungsgemäßen Verbindungen (in DMSO in einer Endkonzentration von 1% [v/v]) mit 15 µg der Rattenlebermikrosomen in 200µl Assaypuffer (6 mM/l MgCl₂, 250 mM/l Saccharose, 150 mM/l KCl, 40 mM/l NaF, 100 mM/l Na₂HPO₄ (pH7,4), 1,3 mM/l ATP, 1,5 mM/l reduziertes Glutathion, 60 uM/l CoA, 0,33 mM/l Nicotinamid und 0,94 mM/l NADH) 10 min bei Raumtemperatur inkubiert. 0,5 µCi [1-¹⁴C]-Stearinsäure (55 mCi/mmol) wurden zugegeben, und die Mischung wurde 1 h bei 37°C inkubiert. Die radioaktiv markierten Fettsäuren wurden anschließend bei 65°C 4 h mit 2,5 M KOH/MeOH:H₂O (4:1) verseift, mit 280 µl Ameisensäure protoniert und mit 500 µl Hexan extrahiert. Die DC-Platten wurden in 10%ige AgNO₃ eingetaucht und vor der Verwendung hitzeaktiviert. 150 µl der Hexanphase wurden auf die Platten aufgebracht, und die DC-Platten wurden in Puffer (Chloroform:Methanol:Essigsäure:Wasser [90:8:1:0,8]) entwickelt und getrocknet. Die Platten wurden zur Quantifizierung der SCD1-Aktivität in einem Phospho-Imager abgelesen.

Der Fachmann kann diesen Assay zur Bestimmung der Inhibierung der Stearoyl-CoA-Desaturase-Aktivität in verschiedener Hinsicht modifizieren. Repräsentative erfindungsgemäße Verbindungen wie die in den Beispielen beschriebenen zeigten, wenn sie in diesem Assay in einer Konzentration von 10 µM/l getestet wurden, Aktivität als Inhibitoren von SCD1, die als Hemmung der SCD1-Aktivität in Prozent angegeben ist.

**Tabelle 2: Biologische Aktivität**

| Bsp. | % Inhibition (10µM) |
|---|---|
| 1 | 65 |
| 2 | 31 |
| 9 | 26 |
| 10 | 100 |
| 13 | 100 |
| 14 | 100 |
| 15 | 100 |
| 16 | 100 |
| 17 | 97 |
| 18 | 38 |
| 19 | 100 |
| 20 | 100 |
| 21 | 100 |
| 22 | 100 |

Die Verbindungen der Formel I hemmen die SCD1-Aktivität und sind daher gut zur Behandlung von Fettstoffwechselstörungen, Obesitas und dem metabolischen Syndrom geignet (Hulver et al. Cell Metabolism (2005), 2(4), 251-261 sowie Warensjoe et al. Diabetologia (2005), 48(10), 1999-2005).

Aufgrund der Hemmung der SCD-Aktivität können die Verbindungen der Formel I auch zur Behandlung bzw. Prävention weiterer durch SCD vermittelten Krankheiten bzw. eines durch SCD vermittelten Leidens in einem Säugetier, vorzugsweise einem Menschen, angewendet werden.

Die Verbindungen der vorliegenden Erfindung eignen sich insbesondere zur Behandlung und/oder Prävention von:
1.- Obesitas, insbesondere viscerale (abdominale) Adipositas
2.-Störungen des Fettsäurestoffwechsels und Glucoseverwertungsstörunge
   - Störungen, bei denen Insulinresistenz eine Rolle spielt
3.- Diabetes mellitus, insbesondere Typ-2-Diabetes, einschließlich der Prävention der damit verbundenen Folgeerkrankungen.
   - Besondere Aspekte in diesem Zusammenhang sind
   - Hyperglykämia,
   - Verbesserung der Insulinresistenz,
   - Verbesserung der Glucosetoleranz,
   - Schutz der β-Zellen der Bauchspeicheldrüse
   - Prävention makro- und mikrovaskulärer Erkrankungen
4.- Dyslipidämien und deren Folgen wie beispielsweise Atherosklerose, koronare Herzkrankheit, zerebrovaskuläre Erkrankungen usw., insbesondere (jedoch nicht darauf beschränkt) die, die durch einen oder mehrere der folgenden Faktoren gekennzeichnet sind:
   - hohe Plasmatriglyceridkonzentrationen, hohe postprandiale Plasmatriglyceridkonzentrationen,
   - niedrige HDL-Cholesterinkonzentration
   - niedrige ApoA-Lipoproteinkonzentrationen
   - hohe LDL-Cholesterinkonzentrationen
   - kleine dichte LDL-Cholesterinpartikel
   - hohe ApoB-Lipoproteinkonzentrationen
   - Desaturierungsindex (z.B. Verhältnis 18:1 / 18:0n-9, 16:1 / 16:0 n-7 oder 18:1n-9 + 16:1n-7 / 16:0 Fettsäuren)
5.- Verschiedene andere leiden, die mit dem metabolischen Syndrom bzw. Syndrom X assoziert sein können, wie
   - Zunahme des Bauchumfangs
   - Dyslipidämie (z.B. Hypertriglyceridämie und/oder niedriges HDL)
   - Insulinresistenz
   - Hyperkoagulabilität
   - Hyperurikämie
   - Mikroalbuminämie
   - Thrombosen, hyperkoagulabile und prothrombotische Zustände (arteriell und venös)
   - Bluthochdruck
   - Herzinsuffizienz, beispielsweise (jedoch nicht darauf beschränkt), nach Herzinfarkt, hypertensiver Herzkrankheit oder Kardiomyopathie
6.- Leberstörungen und damit in Zusammenhang stehende Leiden
   - Fettleber
   - hepatische Steatose
   - nicht alkoholbedingte Hepatitis
   - nicht alkoholbedingte Steatohepatitis (NASH)
   - alkoholbedingte Hepatitis
   - akute Fettleber
   - schwangerschaftsbedingte Fettleber
   - arzneimittelinduzierte Hepatitis
   - Eisenspeicherkrankheiten
   - hepatische Fibrose
   - hepatische Zirrhose
   - Hepatom
   - virale Hepatitis
7.- Hauterkrankungen und -leiden bzw. solche, die mit mehrfach ungesättigten Fettsäuren in Zusammenhang stehen
   - Ekzem
   - Akne
   - Schuppenflechte
   - Bildung bzw. Prävention von Wulstnarben
   - andere mit der Fettsäurezusammensetzung der Schleimhautmembran in Zusammenhang stehende Krankheiten
8.- Primäre Hypertriglyceridämie oder sekundäre Hypertriglyceridämien nach
   - familiärer Retikulohistiozytose
   - Lipoproteinlipasemangel
   - Hyperlipoproteinämien
   - Apolipoproteinmangel (z.B. ApoCII- oder ApoE-Mangel)
9.- Krankheiten bzw. Leiden, die mit neoplastischer zellulärer Proliferation in Zusammenhang stehen
   - gutartige oder maligne Tumore
   - Krebs
   - Neoplasien
   - Metastasen
   - Karzinogenese
10.- Krankheiten bzw. Leiden, die mit neurologischen, psychiatrischen oder Immunerkrankungen bzw. -leiden in Zusammenhang stehen
11.- Andere Krankheiten bzw. Leiden, an denen beispielsweise entzündliche Reaktionen, Zelldifferenzierung und/oder andere durch SCD vermittelte Aspekte beteiligt sein können, sind:
   - Atherosklerose wie beispielsweise (jedoch nicht darauf beschränkt) Koronarsklerose einschließlich Angina pectoris oder Herzinfarkt, Schlaganfall, ischämischer Schlaganfall und transitorische ischämische Attacke (transient ischemic attack, TIA)
   - periphere Verschlußkrankheit
   - vaskuläre Restenose oder Reokklusion
   - chronische entzündliche Darmerkrankungen wie beispielsweise Morbus Crohn und Colitis ulcerosa
   - Pankreatitis
   - Sinusitis
   - andere entzündliche Leiden
   - Retinopathie, ischämische Retinopathie
   - Fettzellentumore
   - lipomatöse Karzinome wie beispielsweise Liposarkome
   - feste Tumore und Neoplasmen wie beispielsweise (jedoch nicht darauf beschränkt) Karzinome des Magen-Darm-Trakts, der Leber, der Gallenwege und der Bauchspeicheldrüse, endokrine Tumore, Karzinome der Lunge, der Niere und der ableitenden Harnwege, des Genitaltrakts, Prostatakarzinome usw.
   - akute und chronische myeloproliferative Erkrankungen und Lymphome
   - Angiogenese
   - neurodegenerative Erkrankungen
   - Alzheimer-Krankheit
   - multiple Sklerose
   - Parkinson-Krankheit
   - erythematosquamöse Dermatosen wie beispielsweise Schuppenflechte
   - Acne vulgaris
   - andere Hauterkrankungen und dermatologische Leiden, die durch PPAR moduliert werden
   - Ekzeme und Neurodermatitis
   - Dermatitis wie beispielsweise seborrhoische Dermatitis oder Lichtdermatitis
   - Keratitis und Keratosen wie beispielsweise seborrhoische Keratosen, senile Keratosen, aktinische Keratoses, lichtinduzierte Keratosen oder Keratosis follicularis
   - Wulstnarben und Wulstnarbenprophylaxe
   - Warzen einschließlich Kondylomen oder Condylomata acuminata
   - Infektionen mit dem humanen Papillomavirus (HPV) wie beispielsweise venerische Papillome, von Viren verursachte Warzen wie beispielsweise Molluscum contagiosum, Leukoplakie
   - papulöse Dermatosen wie beispielsweise Lichen planus
   - Hautkrebs wie beispielsweise Basalzellenkarzinome, Melanome oder kutane T-Zellenlymphome
   - lokalisierte gutartige epidermale Tumore wie beispielsweise Keratoderma, epidermale Naevi
   - Frostbeulen
   - Bluthochdruck
   - Syndrom X
   - Symdrom der polyzystischen Ovarien (polycystic ovary syndrome, PCOS)
   - Asthma
   - zystische Fibrose
   - Osteoarthritis
   - Lupus erythematodes (LE) oder entzündliche rheumatische Erkrankungen wie beispielsweise rheumatoide Arthritis
   - Gefäßentzündung
   - Auszehrung (Kachexie)
   - Gicht
   - Ischämia/Reperfusionssyndrom
   - Schocklunge (acute respiratory distress syndrome, ARDS)
   - Virenerkrankungen und -infektionen
   - Lipodystrophie und lipodystrophische Leiden, auch bei Arzneimittelnebenwirkungen (z.B. nach der Einnahme von Medikamenten zur Behandlung von HIV oder Tumoren)
   - Myopathien und Lipidmyopathien (wie Carnitinpalmitoyltransferase-I- oder -II-Mangel) Bildung von Muskeln und einem schlanken Körper bzw. Muskelmassebildung in der Tierhaltung und beim Menschen

### Darstellung

Die erfindungsgemäßen Verbindungen der allgemeinen Formel I werden durch an sich in der Literatur bekannte Verfahren dargestellt und sind nach den folgenden Vorschriften erhältlich, worin die Reste die oben angegeben Bedeutungen haben:

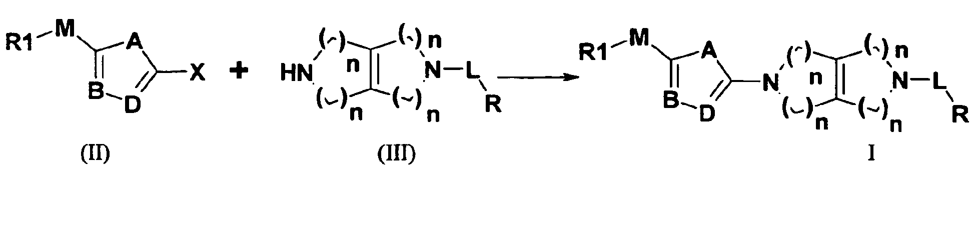

Das Halogenatom in der Heterocycleneinheit (II) wurde durch Austausch mit der Amineinheit ersetzt, worauf beispielsweise die Gruppe M-R1 noch weiter modifiziert werden kann. Falls M-R1 eine Estergruppe darstellt, kann diese verseift werden und die so erhaltenen Carbonsäuren mit Aminen gekuppelt werden. Um die Gruppe L-R der Amineinheit zu modifizieren, stellt man geeigneterweise die Reaktionssequenz um und synthetisiert eine gemeinsame Komponente (IV) und führt die verschiedenen Substituenten im letzten Schritt ein. 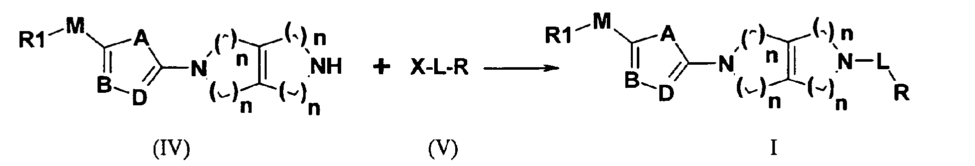 Da bei diesen Umsetzungen gewöhnlich Säuren freigesetzt werden, ist es vorteilhaft, zur Beschleunigung Basen wie Pyridin, Triethylamin, Natronlauge oder Alkalicarbonate zuzusetzen. Die Umsetzungen können in weiten Temperaturbereichen durchgeführt werden. Es hat sich als vorteilhaft erwiesen, bei Temperaturen von 0°C bis zum Siedepunkt des verwendeten Lösungsmittels zu arbeiten. Beispiele von verwendeten Lösungsmitteln sind Methylenchlorid, THF, DMF, Toluol, Essigsäureethylester, n-Heptan, Dioxan, Diethylether oder Pyridin. Unter wasserfreien Bedingungen haben sich auch starke Basen wie Lithiumhydrid, Natriumhydrid oder Kalium-tert.-butanolat in aprotischen Lösungsmitteln wie THF oder DMF als geeignet erwiesen.

Die als Ausgangsmaterialien verwendeten Verbindungen sind kommerziell erhältlich oder lassen sich nach aus der Literatur bekannten Verfahren herstellen; 2-substituierte Thiazol-4-carbonsäureester beispielsweise kann man synthetisieren, indem man die entsprechenden Carbothioamide mit BrCH₂COCO₂Et (analog Sandoz-Patent-GmbH, DE 3443698) cyclokondensiert. Eine alternative Methode ist die Substitution des Bromatoms von 2-Brom-4-thiazolcarbonsäureethylester durch Aminoderivate (analog R.A. Stokbroekx, G.A.J. Grauwels, M. Willems, EP 398425; K. Schiemann, H. Boettcher, H.T. Henning; G. Hoelzemann, C. van Amsterdam, G. Bartoszyk, J. Leibrock, C. Seyfried, WO 2004041815). Die entsprechenden 5-substituierten Derivate lassen sich analog erhalten (s. K. Anandan, X. Xiao, D. V. Patel, J. S. Ward, US 2005250784).

Die Verbindungen der allgemeinen Formel I werden aus dem Reaktionsgemisch isoliert und nach an sich bekannten Verfahren wie Extraktion, Kristallisation oder Chromatographie aufgereinigt. Die nachfolgend aufgeführten Beispiele dienen zur Erläuterung der Erfindung, ohne diese jedoch einzuschränken.
Die Identität der Verbindungen wurde durch Massenspektrometrie geprüft.

### Beispiel 1:

2-[5-(2-Trifluormethyl-benzoyl)-3,4,5,6-tetrahydro-1H-pyrrolo[3,4-c]pyrrol-2-yl]-thiazol-5-carbonsäureethylester

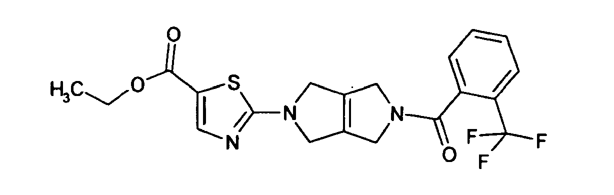

### 1a: 2-(3,4,5,6-Tetrahydro-1H-pyrrolo[3,4-c]pyrrol-2-yl)-thiazol-5- carbonsäureethylester; Trifluoracetat

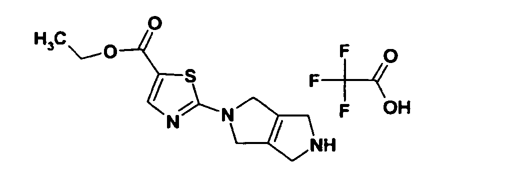

Zu 1,2,3,4,5,6-Hexahydro-pyrrolo[3,4-c]pyrrol-dihydrobromid (1,15 g, 4,24 mmol) und Triethylamin (2,18 ml, 15,67 mmol) in 10 ml DMF wurden bei 80 °C 2-Brom-thiazol-5-carbonsäureethylester (1 g, 4,24 mmol) in 3 ml DMF zugetropft. Nach 3 h bei 80 °C wurde abgekühlt, vom Feststoff abfiltriert, das Filtrat eingeengt und durch präparative HPLC (PR18, Acetonitril/Wasser 0,1% TFA) aufgereinigt. Ausbeute: 60 mg (4%), M+H+: 266,09.

### 1b: 2-[5-(2-Trifluormethyl-benzoyl)-3,4,5,6-tetrahydro-1H-pyrrolo[3,4-c]pyrrol-2-yl]-thiazol-5-carbonsäureethylester

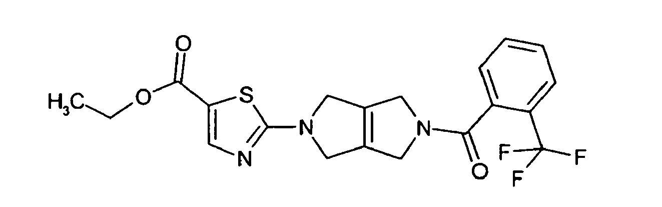

2-Trifluormethyl-benzoylchlorid (50,4 mg, 0,24 mmol) wurden bei RT zu 2-(3,4,5,6-Tetrahydro-1H-pyrrolo[3,4-c]pyrrol-2-yl)-thiazol-5-carbonsäureethylester; TrifluoressigsäureSalz (60 mg, 0,16 mmol) und Triethylamin (64µl, 0,46 mmol) in 4 ml Methylenchlorid und 2 ml Pyridin gegeben. Nach 3 h bei RT wurde eingeengt, Wasser und Essigsäureethylester zugegeben und die organische Phase wurde abgetrennt, eingedampft und durch präparative HPLC (PR18, Acetonitril/Wasser 0,1% TFA) aufgereinigt. Ausbeute:
63 mg (91%), M+H+: 438,04.

### Beispiel 2:

2-[5-(2-Trifluormethyl-benzoyl)-3,4,5,6-tetrahydro-1H-pyrrolo[3,4-c]pyrrol-2-yl]-thiazol-5-carbonsäure (2-cyclopropyl-ethyl)-amid

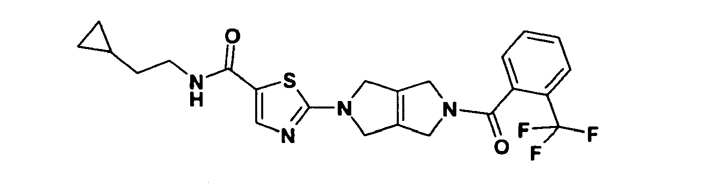

### 2a: 2-[5-(2-Trifluormethyl-benzoyl)-3,4,5,6-tetrahydro-IH-pyrrolo[3,4-c]pyrro1-2-yl]-thiazol-5-carbonsäure, Lithiumsalz

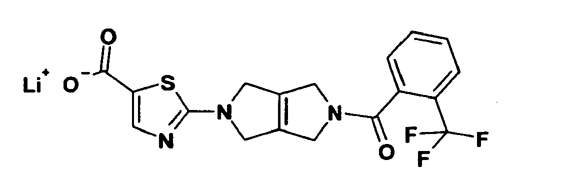

2-[5-(2-Trifluormethyl-benzoyl)-3,4,5,6-tetrahydro-1H-pyrrolo[3,4-c]pyrrol-2-yl]-thiazol-5-carbonsäureethylester (80 mg, 0,18 mmol) und Lithiumhydroxid-hydrat (29 mg, 0,7 mmol) wurden in 10 ml THF und 1 ml Wasser 7 h bei Raumtemperatur gerührt, eingeengt, mit Wasser versetzt und mit Essigsäureethylester extrahiert. Die Wasserphase wurde gefriergetrocknet und direkt weiter umgesetzt.

### 2b: 2-[5-(2-Trifluormethyl-benzoyl)-3,4,5,6-tetrahydro-1H-pyrrolo[3,4-c]pyrrol-2-yl]-thiazol-5-carbonsäure (2-cyclopropyl-ethyl)-amid

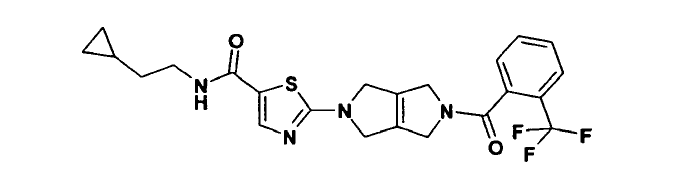

2-[5-(2-Trifluormethyl-benzoyl)-3,4,5,6-tetrahydro-1H-pyrrolo[3,4-c]pyrrol-2-yl]-thiazol-5-carbonsäure, Lithiumsalz (88 mg, 0,212 mmol), 2-Cyclopropyl-ethylamin-hydrochlorid (38,7 mg, 0,318 mmol), Triethylamin (126 µl, 0,92 mmol) und HATU (128 mg, 0,37 mmol) wurden 2 h bei Raumtemperatur gerührt. Das Lösungsmittel wurde abgedampft und der Rückstand durch präparative HPLC (PR18, Acetonitril/Wasser 0,1% TFA) aufgereinigt. Ausbeute: 6 mg (6 %),M+H+:477,19.

### Beispiel 3:

2-[5-(2-Trifluormethyl-benzoyl)-3,4,5,6-tetrahydro-1H-pyrrolo[3,4-c]pyrrol-2-yl]-thiazol-5-carbonsäure-(3-methyl-butyl)-amid

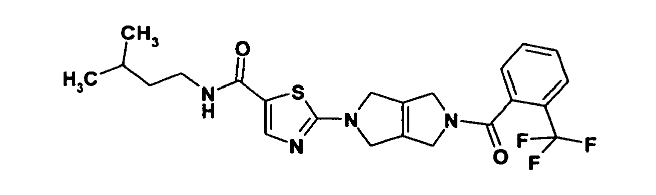

### 3a: 3,4,5,6-Tetrahydro-1H-pyrrolo[3,4-c]pyrrol-2-carbonsäure-tert-butylester

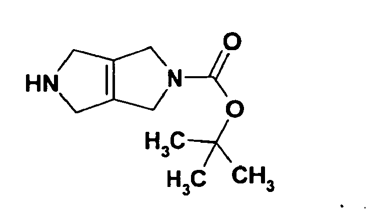

1,2,3,4,5,6-Hexahydro-pyrrolo[3,4-c]pyrrol-dihydrobromid (4,3 g, 15,82 mmol) werden in 215 ml Wasser gelöst und anschließend Natriumhydrogencarbonat (3.45 g , 41,07 mmol) zugegeben. Unter Rühren werden Di-tert-butyl-dicarbonat (3,46 g, 15,85 mmol) in 40 ml Methanol zugegeben und 4 h bei Raumtemperatur gerührt. Der ausgefallene Niederschlag (4,6-Dihydro-1H,3H-pyrrolo[3,4-c]pyrrol-2,5-dicarbonsäure-di-tert-butylester) wird abgesaugt, das Filtrat eingeengt und lyophilisiert. Das Rohprodukt (enthält ca. 2,3 g Produkt) wird direkt weiter umgesetzt.

### 3b: 5-(5-Ethoxycarbonyl-thiazol-2-yl)-3,4,5,6-tetrahydro-1H-pyrrolo[3,4-c]pyrrol-2-carbonsäure-tert-butylester

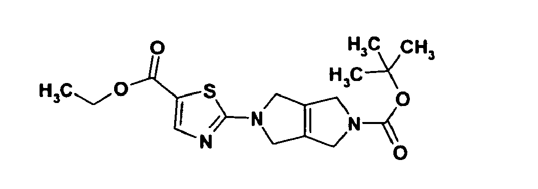

3,4,5,6-Tetrahydro-1H-pyrrolo[3,4-c]pyrrol-2-carbonsäure-tert-butylester (1,2 g, 5,7 mmol), 2-Brom-thiazol-5- carbonsäureethylester (1,69 g, 7,17 mmol) und Trietylamin (1,09 ml, 7,84 mmol) wurden in 70 ml DMF 7 h bei 100 °C gerührt. Der Rückstand wurde abfiltriert, das Filtrat eingeengt und der verbleibende Rückstand mit Essigsäureethylester extrahiert. Man erhielt 1,34 g Rohprodukt, das ohne weitere Reinigung umgesetzt wurde (M+H+: 366,14).

### 3c: 5-(5-Carboxy-thiazol-2-yl)-3,4,5,6-tetrahydro-1H-pyrrolo[3,4-c]pyrrol-2-carbonsäure-tert-butylester

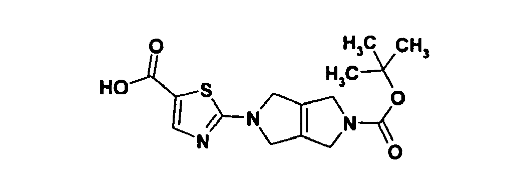

5-(5-Ethoxycarbonyl-thiazol-2-yl)-3,4,5,6-tetrahydro-1H-pyrrolo[3,4-c]pyrrol-2-carbonsäure-tert-butylester (1,34 g, 3,66 mmol) und Lithiumhydroxid-hydrat (385 mg, 9,17 mmol) wurden in 150 ml THF und 50 ml Wasser 2 h bei 50 °C und 8 h bei 80 °C gerührt. THf wurde im Vakuum eingeengt und die Wasserphase mit Essigsäureethylester extrahiert. Die Wasserphase wurde angesäuert und erneut mit Essigsäureethylester extrahiert. Die organische Phase wurde eingeengt. Ausbeute: 662 mg (53 %), M+H+: 338,10.

### 3d: 5-[5-(3-Methyl-butylcarbamoyl)-thiazol-2-yl]-3,4,5,6-tetrahydro-1H-pyrrolo[3,4-c]pyrrol-2-carbonsäure-tert-butylester

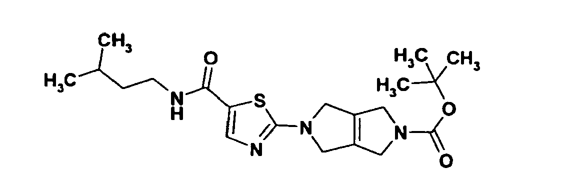

5-(5-Carboxy-thiazol-2-yl)-3,4,5,6-tetrahydro-1H-pyrrolo[3,4-c]pyrrol-2-carbonsäure-tert-butylester (186 mg, 0,55 mmol), Isoamylamin (0,42 ml, 2,6 mmol), Triethylamin (153 µl, 1,1 mmol) und HATU (209 mg, 0,55 mmol) wurden in 20 ml DMF 2 h bei Raumtemperatur gerührt. Das Lösungsmittel wurde abgedampft und der Rückstand mit Wasser und Essigsäureethylester versetzt. Die organische Phase wurde abgetrennt, eingeengt und das Rohprodukt direkt weiter umgesetzt. Ausbeute: 225 mg, M+H+: 407,20.

### 3e: 2-(3,4,5,6-Tetrahydro-1H-pyrrolo[3,4-c]pyrrol-2-yl)-thiazol-5-carbonsäure-(3-methylbutyl)-amid; Trifluoracetat

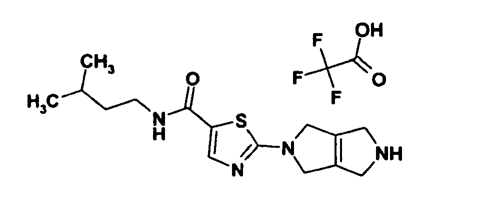

5-[5-(3-Methyl-butylcarbamoyl)-thiazol-2-yl]-3,4,5,6-terahydro-1H-pyrrolo[3,4-c]pyrrol-2-carbonsäure-tert-butylester (225 mg, 0,55 mmol) wurden mit 5 ml Wasser und 0,23 ml Trifluoressigsäure 3 h bei Raumtemperatur gerührt. Nach dem Einengen wird der Rückstand mit Wasser versetzt, mit Essigsäureethylester extrahiert und die Wasserphase lyophilisiert. Ausbeute: 440 mg Rohprodukt, enthält noch Überschuß an Trifluoressigsäure.

### 3f: 2-[5-(2-Trifluormethyl-benzoyl)-3,4,5,6-tetrahydro-1H-pyrrolo[3,4-c]pyrrol-2-yl]-thiazol-5-carbonsäure-(3-methyl-butyl)-amid

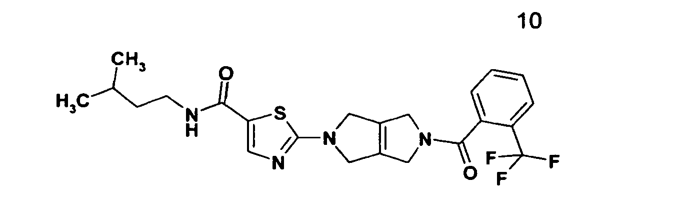

2-(3,4,5,6-Tetrahydro-1H-pyrrolo[3,4-c]pyrrol-2-yl)-thiazol-5-carbonsäure-(3-methyl-butyl)-amid, Trifluoracetat (220 mg, 0,52 mmol) und Triethylamin (0,22 ml, 1,57 mmol) wurden in 10 ml DMF gelöst und im Eisbad mit 2-Trifluormethyl-benzoylchlorid (218 mg, 1,04 mmol) versetzt. Es wurde 1 h weiter gerührt und auf Raumtemperatur erwärmen lassen, eingeengt, Wasser und Essigsäureethylester zugegeben und die organische Phase wurde abgetrennt, eingedampft und durch präparative HPLC (PR18, Acetonitril/Wasser 0,1% TFA) aufgereinigt. Ausbeute: 25 mg (10%), M+H+: 479.06.

### Beispiel 4:

2-[5-(2-Trifluormethyl-benzoyl)-3,4,5,6-tetrahydro-1H-pyrrolo[3,4-c]pyrrol-2-yl]-thiazol-5-carbonsäure-cyclopropylmethyl-amid 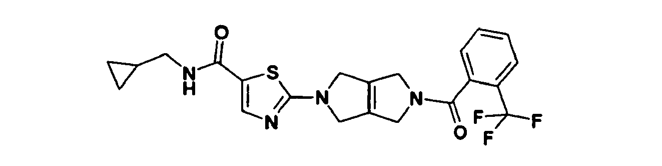 2-(3,4,5,6-Tetrahydro-1H-pyrrolo[3,4-c]pyrrol-2-yl)-thiazole-5-carbonsäure-cyclopropylmethyl-amid, Trifluoracetat (106 mg, 0,26 mmol) wurden analog Beispiel 3f mit 2-Trifluormethyl-benzoylchlorid umgesetzt. M+H+: 463.05.

### Beispiel 5:

2-(5-Chinazolin-4-yl-3,4,5,6-tetrahydro-1H-pyrrolo[3,4-c]pyrrol-2-yl)-thiazol-5-carbonsäure-(3-methyl-butyl)-amid 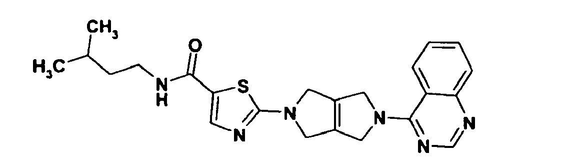 2-(3,4,5,6-Tetrahydro-1H-pyrrolo[3,4-c]pyrrol-2-yl)-thiazol-5-carbonsäure-(3-methyl-butyl)-amid, Trifluoracetat (220 mg, 0,52 mmol) und Triethylamin (0,22 ml, 1,57 mmol) wurden in 10 ml DMF gelöst und im Eisbad mit 4-Chlor-chinazolin (172 mg, 1 mmol) versetzt. Es wurde 1 h weiter gerührt und auf Raumtemperatur erwärmen lassen, eingeengt, Wasser und Essigsäureethylester zugegeben und die organische Phase wurde abgetrennt, eingedampft und durch präparative HPLC (PR18, Acetonitril/Wasser 0,1% TFA) aufgereinigt. M+H+: 435.05.

### Beispiel 6:

2-(5-Chinazolin-4-yl-3,4,5,6-tetrahydro-1H-pyrrolo[3,4-c]pyrrol-2-yl)-thiazol-5- carbonsäurecyclopropylmethyl-amid 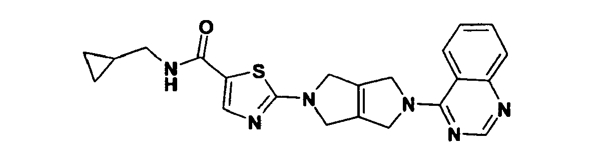 2-(3,4,5,6-Tetrahydro-1H-pyrrolo[3,4-c]pyrrol-2-yl)-thiazol-5-carbonsäure-cyclopropylmethyl-amid, Trifluoracetat (106 mg, 0,26 mmol) wurden analog Beispiel 5 mit 4-Chlor-chinazolin umgesetzt. M+H+: 419.15.

### Beispiel 7:

5-[5-(3-Phenyl-propylcarbamoyl)-thiazol-2-yl]-3,4,5,6-tetrahydro-1H-pyrrolo[3,4-c]pyrrol-2-carbonsäure-tert-butyl ester 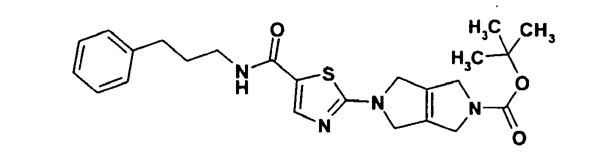 5-(5-Carboxy-thiazol-2-yl)-3,4,5,6-tetrahydro-1H-pyrrolo[3,4-c]pyrrol-2-carbonsäure-tert-butylester wurde analog Beispiel 3d mit 3-Phenylpropylamin umgesetzt. Ausbeute: 91%, M+H+: 455.26.

### Beispiel 8:

2-(5-Chinazolin-4-yl-3,4,5,6-tetrahydro-1H-pyrrolo[3,4-c]pyrrol-2-yl)-thiazol-5- carbonsäure-(3-phenyl-propyl)-amid

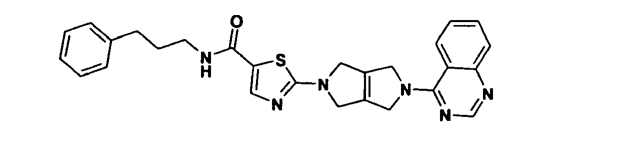

### 8a : 2-(3,4,5,6-Tetrahydro-1H-pyrrolo[3,4-c]pyrrol-2-yl)-thiazol-5- carbonsäure-3-phenylpropyl)-amid; Trifluoracetat

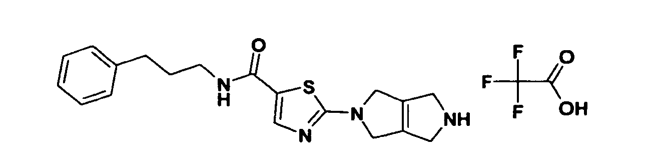

5-[5-(3-Phenyl-propylcarbamoyl)-thiazol-2-yl]-3,4,5,6-tetrahydro-1H-pyrrolo[3,4-c]pyrrol-2-carbonsäure-tert-butyl ester (Beispiel 7) (376 mg, 0,82 mmol) wurden analog Beispiel 3e mit Trifluoressigsäure umgesetzt. Ausbeute: quantitativ, M+H+: 355.10.

### 8b: 2-(5-Chinazolin-4-yl-3,4,5,6-tetrahydro-1H-pyrrolo[3,4-c]pyrrol-2-yl)-thiazol-5-carbonsäure-(3-phenyl-propyl)-amid

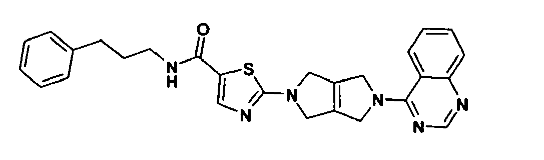

2-(3,4,5,6-Tetrahydro-1H-pyrrolo[3,4-c]pyrrol-2-yl)-thiazol-5-carbonsäure-3-phenyl-propyl)-amid; Trifluoracetat wurde analog Beispiel 5 mit 4-Chlor-chinazolin umgesetzt. Ausbeute: 98 %, M+H+: 483.19.

### Beispiel 9:

2-[5-(2-Trifluormethyl-benzoyl)-3,4,5,6-tetrahydro-1H-pyrrolo[3,4-c]pyrrol-2-yl]-thiazole-5-carbonsäure-(3-phenyl-propyl)-amid 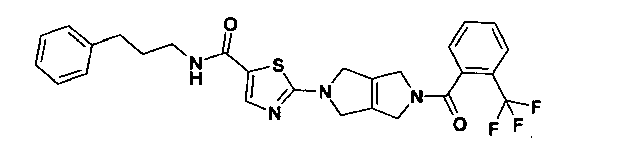 2-(3,4,5,6-Tetrahydro-1H-pyrrolo[3,4-c]pyrrol-2-yl)-thiazol-5- carbonsäure-3-phenyl-propyl)-amid; Trifluoracetat wurde analog Beispiel 3f mit mit 2-Trifluormethyl-benzoylchlorid umgesetzt. Ausbeute: 61 %, M+H+: 527.15.

### Beispiel 10:

N-(3-Methyl-butyl)-6-[5-(2-trifluormethyl-benzoyl)-3,4,5,6-tetrahydro-1H-pyrrolo[3,4-c]pyrrol-2-yl]-nicotinsäureamid

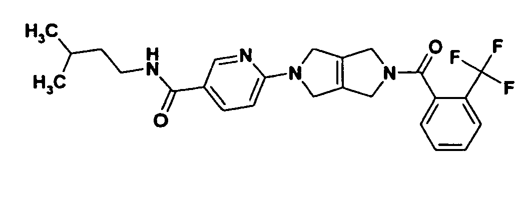

### 10a: 5-(5-Ethoxycarbonyl-pyridin-2-yl)-3,4,5,6-tetrahydro-1H-pyrrolo[3,4-c]pyrrol-2-carbonsäure-tert-butyl ester

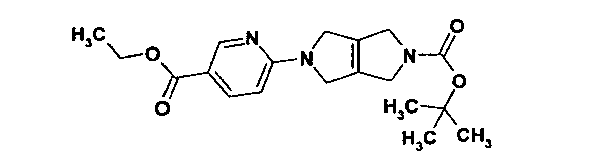

6-Chlor-nicotinsäureethylester (567 mg, 3,05 mmol), 3,4,5,6-Tetrahydro-1H-pyrrolo[3,4-c]pyrrol-2-carbonsäure-tert-butylester (565,9 mg, 2.7 mmol) und Cäsiumcarbonat (878 mg, 2.7 mmol) wurden in 20 ml DMF 8 h bei 100 °C und 8 h bei 120 °C gerührt. Der Rückstand wurde abfiltriert, mit Wasser und Essigsäureethylester gewaschen und getrocknet. Das erste Filtrat wurde eingeengt, mit Essigsäureethylester verrührt und das Produkt abfiltriert. Ausbeute: 72 %, M+H+: 360.20.

### 10b: 6-(3,4,5,6-Tetrahydro-1H-pyrrolo[3,4-c]pyrrol-2-yl)- nicotinsäure-ethylester; Trifluoracetat

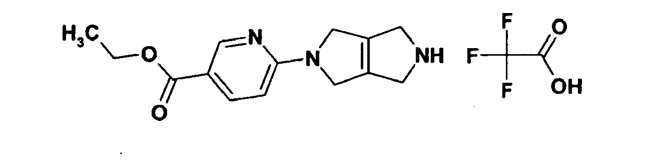

5-(5-Ethoxycarbonyl-pyridin-2-yl)-3,4,5,6-tetrahydro-1H-pyrrolo[3,4-c]pyrrol-2-carbonsäure-tert-butylester (525 mg, 1,46 mmol) wurden anlog Beispiel 3e mit Trifluoressigsäure umgesetzt. Ausbeute: 95 mg Rohprodukt, enthält noch Überschuß an Trifluoressigsäure. M+H+: 260.10.

### 10c: 6-[5-(2-Trifluormethyl-benzoyl)-3,4,5,6-tetrahydro-1H-pyrrolo[3,4-c]pyrrol-2-yl]-nicotinsäure-ethylester

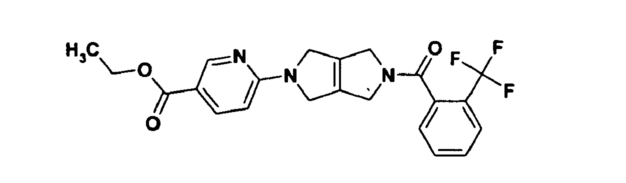

6-(3,4,5,6-Tetrahydro-1H-pyrrolo[3,4-c]pyrrol-2-yl)- nicotinsäure-ethylester; Trifluoracetat (95 mg, 0,25 mmol) und Triethylamin (77, 3 mg, 0,76 mmol) wurden analog Beispiel 3f im Eisbad mit 2-Trifluormethyl-benzoylchlorid (56,6 mg, 0,27 mmol) umgesetzt.
Ausbeute: 99 %, M+H+: 432.10.

### 10d: 6-[5-(2-Trifluormethyl-benzoyl)-3,4,5,6-tetrahydro-1H-pyrrolo[3,4-c]pyrrol-2-yl]-nicotinsäure

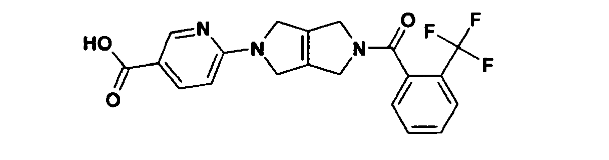

6-[5-(2-Trifluormethyl-benzoyl)-3,4,5,6-tetrahydro-1H-pyrrolo[3,4-c]pyrrol-2-yl]- nicotinsäure-ethylester (110 mg, 0,25 mmol) wurden analog Beispiel 3c umgesetzt. Ausbeute: 40 %, M+H+: 404,10.

### 10e: N-(3-Methyl-butyl)-6-[5-(2-trifluormethyl-benzoyl)-3,4,5,6-tetrahydro-1H-pyrrolo[3,4-c]pyrrol-2-yl]-nicotinsäureamid

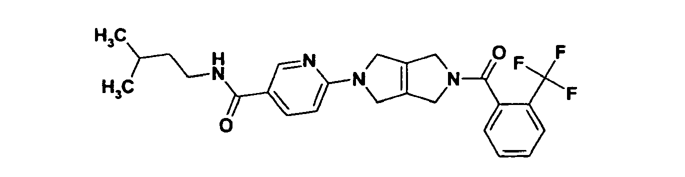

6-[5-(2-Trifluormethyl-benzoyl)-3,4,5,6-tetrahydro-1H-pyrrolo[3,4-c]pyrrol-2-yl]-nicotinsäure (40 mg, 0,099 mmol) wurden analog Beispiel 3d mit Isoamylamin umgesetzt. Ausbeute: 34 %, M+H+: 473.18.

### Beispiel 11:

2-[5-(2-Brom-5-methoxy-benzoyl)-3,4,5,6-tetrahydro-1H-pyrrolo[3,4-c]pyrrol-2-yl]-thiazol-5-carbonsäure-(3-methyl-butyl)-amid 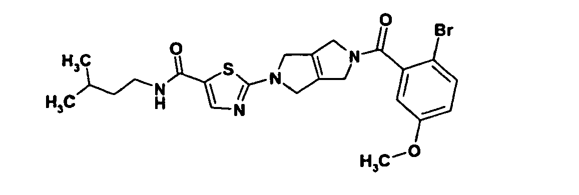 2-(3,4,5,6-Tetrahydro-1H-pyrrolo[3,4-c]pyrrol-2-yl)-thiazol-5-carbonsäure-(3-methyl-butyl)-amid, Trifluoracetat (43,8 mg, 0,1 mmol) wurden analog Beispiel 3f mit 2-Brom-5-methoxy-benzoesäure nach Aktivierung mit HATU umgesetzt. M+H+: 519,15.

### Beispiel 12:

2-[5-(2-Brom-5-methoxy-benzoyl)-3,4,5,6-tetrahydro-1H-pyrrolo[3,4-c]pyrrol-2-yl]-thiazol-5-carbonsäure-(3-phenyl-propyl)-amid 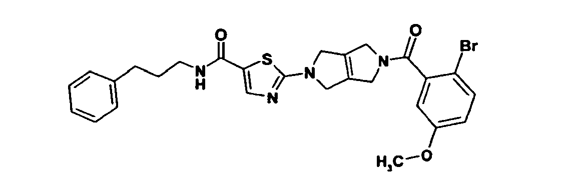 2-(3,4,5,6-Tetrahydro-1H-pyrrolo[3,4-c]pyrrol-2-yl)-thiazol-5-carbonsäure-(3-phenyl-propyl)-amid, Trifluoracetat (44,6 mg, 0,095 mmol) wurden analog Beispiel 3f mit 2-Brom-5-methoxybenzoesäure nach Aktivierung mit HATU umgesetzt. M+H+: 567,17.

### Beispiel 13:

N-Phenethyl-6-[5-(2-trifluorrnethyl-benzoyl)-3,4,5,6-tetrahydro-1H-pyrrolo[3,4-c]pyrrol-2-yl]-nicotinsäureamid 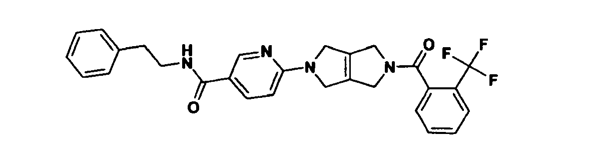 6-[5-(2-Trifluormethyl-benzoyl)-3,4,5,6-tetrahydro-1H-pyrrolo[3,4-c]pyrrol-2-yl]-nicotinsäure (50 mg, 0,124 mmol) wurden analog Beispiel 3d mit 2-Phenylethylamin umgesetzt. Ausbeute: 45 %, M+H+: 507,28.

### Beispiel 14:

N-(2-Cyclopropyl-ethyl)-6-[5-(2-trifluormethyl-benzoyl)-3,4,5,6-tetrahydro-1H-pyrrolo[3,4-c]pyrrol-2-yl]-nicotinsäureamid 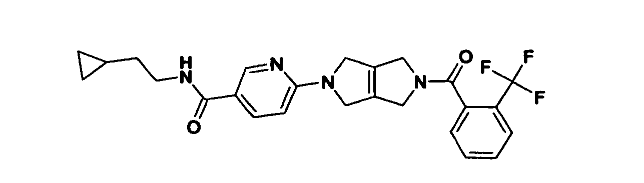 6-[5-(2-Trifluormethyl-benzoyl)-3,4,5,6-tetrahydro-1H-pyrrolo[3,4-c]pyrrol-2-yl]-nicotinsäure (50 mg, 0,124 mmol) wurden analog Beispiel 3d mit 2-Cyclopropylethylamin-Hydrochlorid umgesetzt. Ausbeute: 37 %, M+H+: 471,28.

### Beispiel 15:

N-Thiazol-2-ylmethyl-6-[5-(2-trifluromethyl-benzoyl)-3,4,5,6-tetrahydro-1H-pyrrolo[3,4-c]pyrrol-2-yl]-nicotinsäureamid 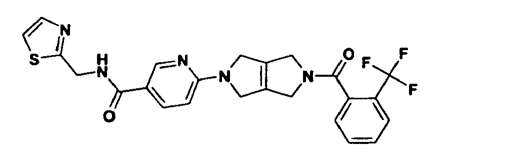 6-[5-(2-Trifluormethyl-benzoyl)-3,4,5,6-tetrahydro-1H-pyrrolo[3,4-c]pyrrol-2-yl]-nicotinsäure (54 mg, 0,134 mmol) wurden analog Beispiel 3d mit C-Thiazol-2-yl-methylamin, Hydrochlorid umgesetzt. Ausbeute: 28 %, M+H+: 500,22.

### Beispiel 16:

N-Methyl-N-thiazol-2-ylmethyl-6-[5-(2-trifluoomethyl-benzoyl)-3,4,5,6-tetrahydro-1H-pyrrolo[3,4-c]pyrrol-2-yl]-nicotinsäureamid 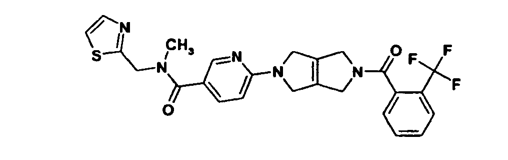 6-[5-(2-Trifluormethyl-benzoyl)-3,4,5,6-tetrahydro-1H-pyrrolo[3,4-c]pyrrol-2-yl]-nicotinsäure (54 mg, 0,134 mmol) wurden analog Beispiel 3d mit Methyl-thiazol-2-ylmethyl-amin umgesetzt. Ausbeute: 52 %, M+H+: 514,30.

### Beispiel 17:

N-Cyclopropylmethyl-6-[5-(2-trifluormethyl-benzoyl)-3,4,5,6-tetrahydro-1H-pyrrolo[3,4-c]pyrrol-2-yl]-nicotinsäureamid 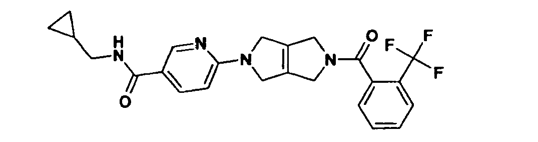 6-[5-(2-Trifluormethyl-benzoyl)-3,4,5,6-tetrahydro-1H-pyrrolo[3,4-c]pyrrol-2-yl]-nicotinsäure (54 mg, 0,134 mmol) wurden analog Beispiel 3d mit C-Cyclopropyl-methylamin umgesetzt. Ausbeute: 77 %, M+H+: 457,26.

### Beispiel 18:

6-[5-(2-Brom-5-methoxy-benzoyl)-3,4,5,6-tetrahydro-1H-pyrrolo[3,4-c]pyrrol-2-yl]-pyridazin-3-carbonsäure-(3-hydroxy-pentyl)-amid 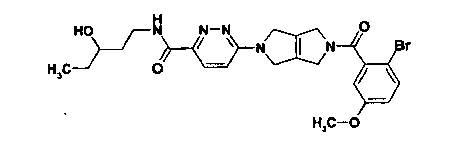 6-(3,4,5,6-Tetrahydro-1H-pyrrolo[3,4-c]pyrrol-2-yl)-pyridazin-3-carbonsäure-(3-hydroxy-pentyl)-amid, Trifluoracetat (466 mg, 0,464 mmol) wurden analog Beispiel 3f mit 2-Brom-5-methoxybenzoesäure nach Aktivierung mit HATU umgesetzt. M+H+: 530,02.

### Beispiel 19:

6-[5-(2-Trifluormethyl-benzoyl)-3,4,5,6-tetrahydro-1H-pyrrolo[3,4-c]pyrrol-2-yl]-pyridazin-3-carbonsäure-(2-cyclopropyl-ethyl)-amid 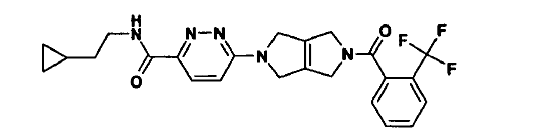 6-Chlor-pyridazin-3-carbonsäure-(2-cyclopropyl-ethyl)-amid (150 mg, 0,665 mmol) und (3,4,5,6-Tetrahydro-1H-pyrrolo[3,4-c]pyrrol-2-yl)-(2-trifluormethyl-phenyl)-methanone, Trifluoracetat (263 mg, 0,665 mmol) wurden analog Beispiel 3b umgesetzt. Ausbeute: 27 %, M+H+: 472,08.

### Beispiel 20:

N-Thiazol-5-ylmethyl-6-[5-(2-trifluormethyl-benzoyl)-3,4,5,6-tetrahydro-1H-pyrrolo[3,4-c]pyrrol-2-yl]-nicotinsäureamid 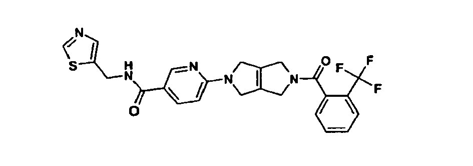 6-[5-(2-Trifluormethyl-benzoyl)-3,4,5,6-tetrahydro-1H-pyrrolo[3,4-c]pyrrol-2-yl]-nicotinsäure (50 mg, 0,124 mmol) wurden analog Beispiel 3d mit C-Thiazol-5-yl-methylamin, Hydrochlorid umgesetzt. Ausbeute: 58 %, M+H+: 500,20.

### Beispiel 21:

N-Cyclopentylmethyl-6-[5-(2-trifluormethyl-benzoyl)-3,4,5,6-tetrahydro-1H-pyrrolo[3,4-c]pyrrol-2-yl]-nicotinsäureamid 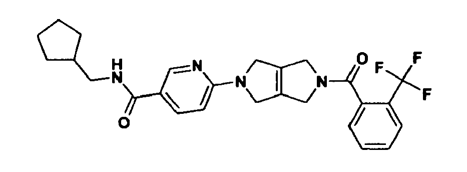 6-[5-(2-Trifluormethyl-benzoyl)-3,4,5,6-tetrahydro-1H-pyrrolo[3,4-c]pyrrol-2-yl]-nicotinsäure (50 mg, 0,124 mmol) wurden analog Beispiel 3d mit C-Cyclopentyl-methylamin, Hydrochlorid umgesetzt. Ausbeute: 69 %, M+H+: 485,28.

### Beispiel 22:

N-(2-Cyclopentyl-ethyl)-6-[5-(2-trifluormethyl-benzoyl)-3,4,5,6-tetrahydro-1H-pyrrolo[3,4-c]pyrrol-2-yl]-nicotinsäureamid 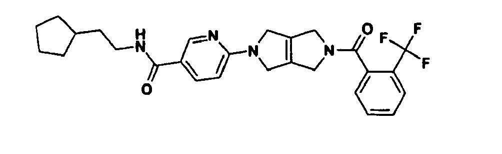 6-[5-(2-Trifluormethyl-benzoyl)-3,4,5,6-tetrahydro-1H-pyrrolo[3,4-c]pyrrol-2-yl]-nicotinsäure (50 mg, 0,124 mmol) wurden analog Beispiel 3d mit Cyclopentyl-ethylamin umgesetzt. Ausbeute: 34 %, M+H+: 499,28.

### Beispiel 23:

N-Thiazol-4-ylmethyl-6-[5-(2-trifluormethyl-benzoyl)-3,4,5,6-tetrahydro-1H-pyrrolo[3,4-c]pyrrol-2-yl]-nicotinsäureamid 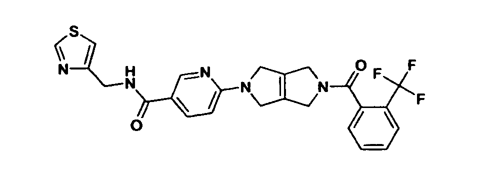 6-[5-(2-Trifluormethyl-benzoyl)-3,4,5,6-tetrahydro-1H-pyrrolo[3,4-c]pyrrol-2-yl]-nicotinsäure (140 mg, 0,347 mmol) wurden analog Beispiel 3d mit C-Thiazol-4-yl-methylamin, Hydrochlorid umgesetzt. Ausbeute: 16 %, M+H+: 500,40.

### Beispiel 24:

2-[5-(2-Trifluoromethyl-benzoyl)-3,4,5,6-tetrahydro-1H-pyrrolo[3,4-c]pyrrol-2-yl]-pyrimidine-5-carboxylic acid (2-cyclopropyl-ethyl)-amide 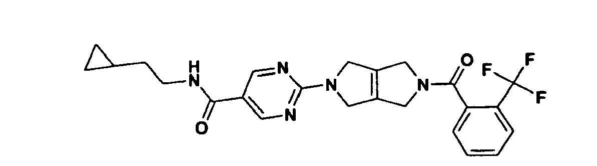 2-(3,4,5,6-Tetrahydro-1H-pyrrolo[3,4-c]pyrrol-2-yl)-pyrimidine-5-carboxylic acid (2-cyclopropyl-ethyl)-amide; compound with trifluoro-acetic acid (300 mg, 0,726 mmol) wurden analog Beispiel 3f mit 2-Trifluormethyl-benzoylchlorid umgesetzt. Ausbeute: 23 %, M+H+: 472,16.

## Patentansprüche

1. Verbindungen der Formel I, 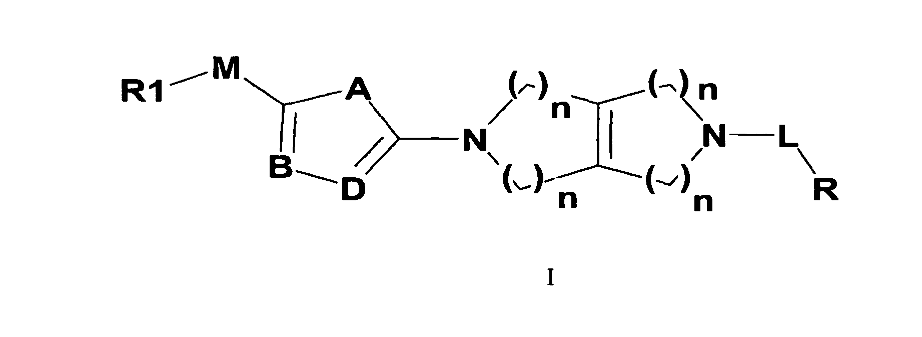 worin bedeuten
R Wasserstoff, (C₁-C₁₆)-Alkyl, (C₁-C₅)-Alkyloxy, (C₁-C₅)-Alkylthio, (C₁-C₅)- Alkylamino, Di-(C₂-C₈)-alkylamino, (C₀-C₄)-Alkylen-(C₆-C₁₀)-aryl, (C₀-C₄)- Alkylen-(C₅-C₁₂)-heteroaryl, (C₀-C₄)-Alkylen-(C₃-C₁₂)-heterocyclyl, (C₀-C₄)- Alkylen-(C₃-C₁₂)-cycloalkyl, ein bicyclisches (C₈-C₁₄)-Ringsystem, wobei Aryl, Heteroaryl, Heterocyclyl Cycloalkyl oder das bicyclisches (C₈-C₁₄)-Ringsystem, ein- oder mehrfach mit Halogen, (C₁-C₆)-Alkyl, (C₁-C₃)-Alkyloxy, Hydroxy, (C₁-C₆)-Alkylmercapto, Amino, (C₁-C₆)- Alkylamino, Di-(C₂-C₁₂)-alkylamino, Mono-(C₁-C₆)- alkylaminocarbonyl, Di-(C₂-C₈)-alkylaminocarbonyl, (C₁-C₆)- Alkoxycarbonyl, (C₁-C₆)-Alkylcarbonyl, Cyano, Trifluormethyl, Trifluormethyloxy, (C₁-C₆)-Alkylsulfonyl oder Aminosulfonyl substituiert sein können;
R1 Wasserstoff, (C₁-C₁₀)-Alkyl, (C₁-C₁₀)-Alkyloxy, Amino, Mono-(C₁-C₁₀)- Alkylamino, Di-(C₂-C₁₂)-alkylamino, wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- , Alkylamino, Di-(C₂-C₁₂)-alkylamino, -(C₆-C₁₀)-Aryl, -(C₅-C₁₂)- Heteroaryl, -(C₃-C₁₂)-Heterocyclyl oder -(C₃-C₁₂)-Cycloalkyl, wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
R2 Wasserstoff, (C₁-C₁₆)-Alkyl, (C₀-C₄)-Alkylen-(C₆-C₁₀)-aryl;
R3 Wasserstoff, (C₁-C₆)-Alkyl, (C₁-C₃)-Alkyloxy, Hydroxy, (C₁-C₆)-Alkyl- mercapto, Amino, (C₁-C₆)-Alkylamino, Di-(C₂-C₁₂)-aikylamino, Cyano, (C₁- C₆)-Alkylcarbonyl, Halogen, Trifluormethyl, Trifluormethyloxy, (C₁-C₆)- Alkylsulfonyl, Aminosulfonyl;
A O, S, N(R2), C(R3), C(R3)=C(R3), NH=C(R3), C(R3)=NH, NH=NH;
B C(R3), N(R2);
D C(R3), N(R2);
wobei mindestens eines der Glieder A, B oder D Stickstoff sein muss;
n unabhängig voneinander jeweils 1 oder 2;
L eine Bindung, -C(=O)-, -C(=S)-, -C(=O)-N(R2)-, -C(=O)-O-, -S(O)₀₋₂-, -S(O)₀₋₂-N(R2)-, ein mono- oder bicyclisches Ringsystem, worin ein oder mehrere Ringglieder N(R3), O S oder -C(=O)- sein können;
M -C(=O)-N(R2)-, -N(R2)-C(=O)-, -S(O)₀₋₂-, -S(O)₀₋₂-N(R2)-, -N(R2)-S(O)₀₋₂-, -C(=O)-O-, -O-C(=O)-;
wobei der Begriff "Cycloalkyl" jeweils für ein gesättigtes oder teilweise ungesättigtes Ringsystem steht, das ausschließlich Kohlenstoffatome enthält und das ein oder mehrere Ringe umfasst
und deren physiologisch verträgliche Salze.

2. Verbindungen der Formel I, gemäß Anspruch 1, **dadurch gekennzeichnet, dass** darin bedeuten
R Wasserstoff, (C₁-C₁₆)-Alkyl, (C₁-C₅)-Alkyloxy, (C₁-C₅)-Alkylthio, (C₁-C₅)- Alkylamino, Di-(C₂-C₈)-alkylamino, (C₀-C₄)-Alkylen-(C₆-C₁₀)-aryl, (C₀-C₄)- Alkylen-(C₅-C₁₂)-heteroaryl, (C₀-C₄)-Alkylen-(C₃-C₁₂)-heterocyclyl, (C₀-C₄)- Alkylen-(C₃-C₁₂)-cycloalkyl, ein bicyclisches (C₈-C₁₄)-Ringsystem, wobei Aryl, Heteroaryl, Heterocyclyl Cycloalkyl oder das bicyclisches (C₈-C₁₄)-Ringsystem, ein- oder mehrfach mit Halogen, (C₁-C₆)-Alkyl, (C₁-C₃)-Alkyloxy, Hydroxy, (C₁-C₆)-Alkylmercapto, Amino, (C₁-C₆)- Alkylamino, Di-(C₂-C₁₂)-alkylamino, Mono-(C₁-C₆)-alkylaminocarbonyl, Di-(C₂-C₈)-alkylaminocarbonyl, (C₁-C₆)-Alkoxycarbonyl, (C₁-C₆)- Alkylcarbonyl, Cyano, Trifluormethyl, Trifluormethyloxy, (C₁-C₆)- Alkylsulfonyl oder Aminosulfonyl substituiert sein können;
R1 Wasserstoff, (C₁-C₁₀)-Alkyl, (C₁-C₁₀)-Alkyloxy, Amino, Mono-(C₁-C₁₀)- Alkylamino, Di-(C₂-C₁₂)-alkylamino, wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₅-C₁₂)- Heteroaryl, -(C₃-C₁₂)-Heterocyclyl oder -(C₃-C₁₂)-Cycloalkyl, wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
R2 Wasserstoff, (C₁-C₁₆)-Alkyl, (C₀-C₄)-Alkylen-(C₆-C₁₀)-aryl;
R3 Wasserstoff, (C₁-C₆)-Alkyl, (C₁-C₃)-Alkyloxy, Hydroxy, (C₁-C₆)-Alkylmercapto, Amino, (C₁-C₆)-Alkylamino, Di-(C₂-C₁₂)-alkylamino, Cyano, (C₁-C₆)- Alkylcarbonyl, Halogen, Trifluorinethyl, Trifluormethyloxy, (C₁-C₆)- Alkylsulfonyl, Aminosulfonyl;
A O, S, N(R2), C(R3), C(R3)=C(R3);
B C(R3), N(R2);
D C(R3), N(R2);
wobei mindestens eines der Glieder A, B oder D Stickstoff sein muss;
n unabhängig voneinander jeweils 1 oder 2;
L eine Bindung, -C(=O)-, -C(=S)-, -C(=O)-N(R2)-, -C(=O)-O-, -S(O)₀₋₂-, -S(O)₀₋₂-N(R2)-, ein mono- oder bicyclisches Ringsystem, worin ein oder mehrere Ringglieder N(R3), O, S oder -C(=O)- sein können;
M -C(=O)-N(R2)-, -N(R2)-C(=O)-, -S(O)₀₋₂-, -S(O)₀₋₂-N(R2)-, -N(R2)-S(O)₀₋₂-, -C(=O)-O-, -O-C(=O)-;
und deren physiologisch verträgliche Salze.

3. Verbindungen der Formel I, gemäß Anspruch 1 oder 2, **dadurch gekennzeichnet, dass** darin bedeuten
n 1;
R Wasserstoff, (C₁-C₁₆)-Alkyl, (C₁-C₅)-Alkyloxy, (C₁-C₅)-Alkylthio, (C₁-C₅)- Alkylamino, Di-(C₂-C₈)-alkylamino, (C₀-C₄)-Alkylen-(C₆-C₁₀)-aryl, (C₀-C₄)- Alkylen-(C₅-C₁₂)-heteroaryl, (C₀-C₄)-Alkylen-(C₃-C₁₂)-heterocyclyl, (C₀-C₄)- Alkylen-(C₃-C₁₂)-cycloalkyl, ein bicyclisches (C₈-C₁₄)-Ringsystem, wobei Aryl, Heteroaryl, Heterocyclyl, Cycloalkyl oder das bicyclisches (C₈-C₁₄)-Ringsystem, ein- oder mehrfach mit Halogen, (C₁-C₆)-Alkyl, (C₁-C₃)-Alkyloxy, Hydroxy, (C₁-C₆)-Alkylmercapto, Amino, (C₁-C₆)- Alkylamino, Di-(C₂-C₁₂)-alkylamino, Mono-(C₁-C₆)-alkylaminocarbonyl, Di-(C₂-C₈)-alkylaminocarbonyl, (C₁-C₆)-Alkoxycarbonyl, (C₁-C₆)- Alkylcarbonyl, Cyano, Trifluormethyl, Trifluormethyloxy, (C₁-C₆)- Alkylsulfonyl oder Aminosulfonyl substituiert sein können;
R1 (C₁-C₁₀)-Alkyl, (C₁-C₁₀)-Alkyloxy, Amino, Mono-(C₁-C₁₀)-Alkylamino, Di-(C₂- C₁₂)-alkylamino, wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₅-C₁₂)- Heteroaryl, -(C₃-C₁₂)-Heterocyclyl oder -(C₃-C₁₂)-Cycloalkyl, wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
R2 Wasserstoff, (C₁-C₁₆)-Alkyl, (C₀-C₄)-Alkylen-(C₆-C₁₀)-aryl;
R3 Wasserstoff, (C₁-C₆)-Alkyl, (C₁-C₃)-Alkyloxy, Hydroxy, (C₁-C₆)-Alkylmercapto, Amino, (C₁-C₆)-Alkylamino, Di-(C₂-C₁₂)-alkylainino, Cyano, (C₁-C₆)- Alkylcarbonyl, Halogen, Trifluormethyl, Trifluormethyloxy, (C₁-C₆)- Alkylsulfonyl, Aminosulfonyl;
A O, S, N(R2), C(R3), C(R3)=C(R3);
B C(R3), N(R2);
D C(R3), N(R2);
wobei mindestens eines der Glieder A, B oder D Stickstoff sein muss;
L eine Bindung, -C(=O)-, -C(=S)-, -C(=O)-N(R2)-, -C(=O)-O-, -S(O)₀₋₂-, -S(O)₀₋₂-N(R2)-, ein mono- oder bicyclisches Ringsystem, worin ein oder mehrere Ringglieder N(R3), O, S oder -C(=O)- sein können;
M -C(=O)-N(R2)-, N(R2)-C(=O)-, -S(O)₀₋₂-, -S(O)₀₋₂-N(R2)-, -N(R2)-S(O)₀₋₂-, -O-C(=O)-;
und deren physiologisch verträgliche Salze.

4. Verbindungen der Formel I, gemäß einem oder mehreren der Ansprüche 1 bis 3, **dadurch gekennzeichnet, dass** darin bedeuten
n 1;
R Wasserstoff, (C₁-C₁₆)-Alkyl, (C₁-C₅)-Alkyloxy, (C₁-C₅)-Alkylthio, (C₁-C₅)- Alkylamino, Di-(C₂-C₈)-alkylamino, (C₀-C₄)-Alkylen-(C₆-C₁₀)-aryl, (C₀-C₄)- Alkylen-(C₅-C₁₂)-heteroaryl, (C₀-C₄)-Alkylen-(C₃-C₁₂)-heterocyclyl, (C₀-C₄)- Alkylen-(C₃-C₁₂)-cycloalkyl, ein bicyclisches (C₈-C₁₄)-Ringsystem, wobei Aryl, Heteroaryl, Heterocyclyl, Cycloalkyl oder das bicyclisches (C₈-C₁₄)-Ringsystem, ein- oder mehrfach mit Halogen, (C₁-C₆)-Alkyl, (C₁-C₃)-Alkyloxy, Hydroxy, (C₁-C₆)-Alkylmercapto, Amino, (C₁-C₆)- Alkylamino, Di-(C₂-C₁₂)-alkylamino, Mono-(C₁-C₆)-alkylaminocarbonyl, Di-(C₂-C₈)-alkylaminocarbonyl, (C₁-C₆)-Alkoxycarbonyl, (C₁-C₆)- Alkylcarbonyl, Cyano, Trifluormethyl, Trifluormethyloxy, (C₁-C₆)- Alkylsulfonyl oder Aminosulfonyl substituiert sein können;
R1 (C₁-C₁₀)-Alkyl, (C₁-C₁₀)-Alkyloxy, Amino, Mono-(C₁-C₁₀)-Alkylamino, Di-(C₂- C₁₂)-alkylamino, wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₅-C₁₂)- Heteroaryl, -(C₃-C₁₂)-Heterocyclyl oder -(C₃-C₁₂)-Cycloalkyl, wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
R2 Wasserstoff, (C₁-C₁₆)-Alkyl, (C₀-C₄)-Alkylen-(C₆-C₁₀)-aryl;
R3 Wasserstoff, (C₁-C₆)-Alkyl, (C₁-C₃)-Alkyloxy, Hydroxy, (C₁-C₆)-Alkylmercapto, Amino, (C₁-C₆)-Alkylarnino, Di-(C₂-C₁₂)-alkylamino, Cyano, (C₁-C₆)- Alkylcarbonyl, Halogen, Trifluormethyl, Trifluormethyloxy, (C₁-C₆)- Alkylsulfonyl, Aminosulfonyl;
A S, C(R3)=C(R3);
B C(R3);
D N(R2);
L eine Binding, -C(=O)-, -C(=S)-, -C(=O)-N(R2)-, -C(=O)-O-, -S(O)₀₋₂-, -S(O)₀₋₂-N(R2)-, ein mono- oder bicyclisches Ringsystem, worin ein oder mehrere Ringglieder N(R3), O, S oder -C(=O)- sein können;
M -C(=O)-N(R2)-, N(R2)-C(=O)-, -S(O)₀₋₂-, -S(O)₀₋₂-N(R2)-, -N(R2)-S(O)₀₋₂-, -O-C(=O)-;
und deren physiologisch verträgliche Salze.

5. Verbindungen der Formel I, gemäß einem oder mehreren der Ansprüche 1 bis 4, **dadurch gekennzeichnet, dass** darin bedeuten
n 1;
R (C₁-C₁₆)-Alkyl, (C₁-C₅)-Alkyloxy, (C₀-C₄)-Alkylen-(C₆-C₁₀)-aryl, ein bicycli- sches (C₈-C₁₄)-Ringsystem, wobei Aryl oder das bicyclisches (C₈-C₁₄)-Ringsystem ein- oder mehrfach mit Halogen, (C₁-C₆)-Alkyl, (C₁-C₃)-Alkyloxy, Hydroxy, (C₁- C₆)-Alkylmercapto, Amino, (C₁-C₆)-Alkylamino, Di-(C₂-C₁₂)- alkylamino, Mono-(C₁-C₆)-alkylaminocarbonyl, Di-(C₂-C₈)- alkylaminocarbonyl, (C₁-C₆)-Alkoxycarbonyl, (C₁-C₆)-Alkylcarbonyl, Cyano, Trifluormethyl, Trifluormethyloxy, (C₁-C₆)-Alkylsulfonyl oder Aminosulfonyl substituiert sein können;
R1 (C₁-C₁₀)-Alkyl, (C₁-C₁₀)-Alkyloxy, Amino, Mono-(C₁-C₁₀)-Alkylamino, Di-(C₂- C₁₂)-alkylamino, wobei Alkyl substituiert sein kann mit Halogen, (C₁-C₆)-Alkyl, (C₁-C₃)- Alkyloxy, Hydroxy, (C₁-C₆)-Alkylmercapto, Amino, (C₁-C₆)- Alkylamino, Di-(C₂-C₁₂)-alkylamino, -(C₆-C₁₀)-Aryl, -(C₅-C₁₂)- Heteroaryl, -(C₃-C₁₂)-Heterocyclyl oder -(C₃-C₁₂)-Cycloalkyl, wobei Aryl, Heteroaryl, Heterocyclyl oder Cycloalkyl gegebenenfalls ein- oder mehrfach durch Halogen, (C₁-C₆)-Alkyl, (C₁-C₃)-Alkyloxy, Hydroxy, (C₁-C₆)-Alkylmercapto, Amino, (C₁- C₆)-Alkylamino, Di-(C₂-C₁₂)-alkylamino, substituiert sein können;
R2 Wasserstoff, (C₁-C₁₆)-Alkyl, (C₀-C₄)-Alkylen-(C₆-C₁₀)-aryl;
R3 Wasserstoff, (C₁-C₆)-Alkyl, (C₁-C₃)-Alkyloxy, Hydroxy, (C₁-C₆)-Alkylmercapto, Amino, (C₁-C₆)-Alkylamino, Di-(C₂-C₁₂)-alkylamino, Cyano, (C₁-C₆)- Alkylcarbonyl, Halogen, Trifluormethyl, Trifluormethyloxy, (C₁-C₆)- Alkylsulfonyl, Aminosulfonyl;
A S, C(R3)=C(R3);
B C(R3);
D N(R2);
L eine Bindung, -C(=O)-;
M -C(=O)-N(R2)-, -O-C(=O)-;
und deren physiologisch verträgliche Salze.

6. Verbindungen gemäß einem oder mehreren der Ansprüche 1 bis 5, zur Anwendung als Arzneimittel.

7. Arzneimittel enthaltend eine oder mehrere der Verbindungen gemäß einem oder mehreren der Ansprüche 1 bis 5.

8. Arzneimittel enthaltend eine oder mehrere der Verbindungen gemäß einem oder mehreren der Ansprüche 1 bis 5 und mindestens einen weiteren Wirkstoff.

9. Arzneimittel, gemäß Anspruch 8, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, PPAR delta Agonisten, Fibrate, MTP-Inhibitoren, Gallensäureresorptionsinhibitoren, MTP-Inhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen Synthetase Inhibitoren, Lipoprotein(a) Antagonisten, HM74A Rezeptor Agonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, Glykogen Phosphorylase Inhibitoren, Glukagon-Rezeptor-Antagonisten, Aktivatoren der Glukokinase, Inhibitoren der Glukoneogenese, Inhibitoren der Fructose-1,6-biphosphatase, Modulatoren des Glukosetransporters-4, Inhibitoren der Glutamin-Fructose-6-Phosphat-Amidotransferase, Inhibitoren der Dipeptidylpeptidase-IV, Hemmstoffe der 11-beta-Hydroxysteroid-Dehydrogenase-1, Inhibitoren der Protein-Tyrosin-Phosphatase-1B, Modulatoren des natrium-abhängigen Glukosetransporters 1 oder 2, Modulatoren des GPR40, Inhibitoren der hormon-sensitiven Lipase, Hemmstoffe der Acetyl-CoA Carboxylase, Inhibitoren der Phosphoenolpyruvatcarboxykinase, Inhibitoren der Glykogen Synthase Kinase-3 beta, Inhibitoren der Protein Kinase C beta, Endothelin-A-Rezeptor Antagonisten, Inhibitoren der I kappaB Kinase, Modulatoren des Glukocorticoidrezeptors, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, , CB1-Rezeptor Antagonisten ,MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

10. Verwendung der Verbindungen gemäß einem oder mehreren der Ansprüche 1 bis 5 zur Herstellung eines Medikamentes zur Behandlung des Diabetes.

11. Verwendung der Verbindungen gemäß einem oder mehreren der Ansprüche 1 bis 5 zur Herstellung eines Medikamentes zur Lipidsenkung.

12. Verwendung der Verbindungen gemäß einem oder mehreren der Ansprüche 1 bis 5 zur Herstellung eines Medikamentes zur Behandlung des metabolischen Syndroms.

13. Verwendung der Verbindungen gemäß einem oder mehreren der Ansprüche 1 bis 5 zur Herstellung eines Medikaments zur Behandlung von Insulin Resistenz.

14. Verwendung der Verbindungen gemäß einem oder mehreren der Ansprüche 1 bis 5 zur Herstellung eines Medikamentes zur Behandlung von Obesitas

15. Verwendung der Verbindungen gemäß einem oder mehreren der Ansprüche 1 bis 5 zur Herstellung eines Medikamentes zur Behandlung von Herzkreislauferkrankungen.

16. Verwendung der Verbindungen gemäß einem oder mehreren der Ansprüche 1 bis 5 zur Herstellung eines Medikamentes zur Behandlung von ZNS-Erkrankungen.

17. Verwendung der Verbindungen gemäß einem oder mehreren der Ansprüche 1 bis 5 zur Herstellung eines Medikamentes zur Behandlung von Schizophrenie.

18. Verwendung der Verbindungen gemäß einem oder mehreren der Ansprüche 1 bis 5 zur Herstellung eines Medikamentes zur Behandlung von Alzheimer.

19. Verfahren zur Herstellung eines Arzneimittels enthaltend eine oder mehrere der Verbindungen gemäß einem oder mehreren der Ansprüche 1 bis 5, **dadurch gekennzeichnet,**
**dass** der Wirkstoff mit einem pharmazeutisch geeigneten Träger vermischt wird und diese Mischung in eine für die Verabreichung geeignete Form gebracht wird.

## Claims

1. A compound of the formula I, 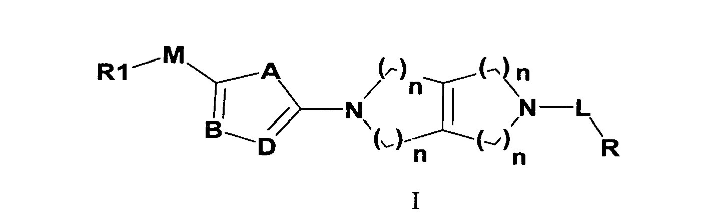 in which
R is hydrogen, (C₁-C₁₆)-alkyl, (C₁-C₅)-alkyloxy, (C₁-C₅)-alkylthio, (C₁-C₅)- alkylamino, di-(C₂-C₈)-alkylamino, (C₀-C₄)-alkylene-(C₆-C₁₀)-aryl, (C₀-C₄)- alkylene-(C₅-C₁₂)-heteroaryl, (C₀-C₄)-alkylene-(C₃-C₁₂)-heterocyclyl, (C₀-C₄)- alkylene-(C₃-C₁₂)-cycloalkyl, a bicyclic (C₈-C₁₄) ring system, where aryl, heteroaryl, heterocyclyl, cycloalkyl or the bicyclic (C₈-C₁₄) ring system may be mono- or polysubstituted by halogen, (C₁-C₆)-alkyl, (C₁-C₃)-alkyloxy, hydroxyl, (C₁-C₆)-alkylmercapto, amino, (C₁-C₆)- alkylamino, di-(C₂-C₁₂)-alkylamino, mono-(C₁-C₆)-alkylamino- carbonyl, di-(C₂-C₈)-alkylaminocarbonyl, (C₁-C₆)-alkoxycarbonyl, (C₁-C₆)-alkylcarbonyl, cyano, trifluoromethyl, trifluoromethyloxy, (C₁-C₆)-alkylsulfonyl or aminosulfonyl;
R1 is hydrogen, (C₁-C₁₀)-alkyl, (C₁-C₁₀)-alkyloxy, amino, mono-(C₁-C₁₀)-alkyl- amino, di-(C₂-C₁₂)-alkylamino, where alkyl may be substituted by halogen, (C₁-C₆)-alkyl, (C₁-C₃)- alkyloxy, hydroxyl, (C₁-C₆)-alkylmercapto, amino, (C₁-C₆)-alkylamino, di-(C₂-C₁₂)-alkylamino, -(C₆-C₁₀)-aryl, -(C₅-C₁₂)-heteroaryl, -(C₃-C₁₂)- heterocyclyl or -(C₃-C₁₂)-cycloalkyl, where aryl, heteroaryl, heterocyclyl or cycloalkyl may each optionally be mono- or polysubstituted by halogen, (C₁-C₆)- alkyl, (C₁-C₃)-alkyloxy, hydroxyl, (C₁-C₆)-alkylmercapto, amino, (C₁-C₆)-alkylamino, di-(C₂-C₁₂)-alkylamino;
R2 is hydrogen, (C₁-C₁₆)-alkyl, (C₀-C₄)-alkylene-(C₆-C₁₀)-aryl;
R3 is hydrogen, (C₁-C₆)-alkyl, (C₁-C₃)-alkyloxy, hydroxyl, (C₁-C₆)-alkylmercapto, amino, (C₁-C₆)-alkylamino, di-(C₂-C₁₂)-alkylamino, cyano, (C₁-C₆)-alkyl- carbonyl, halogen, trifluoromethyl, trifluoromethyloxy, (C₁-C₆)-alkylsulfonyl, aminosulfonyl;
A is O, S, N(R2), C(R3), C(R3)=C(R3), NH=C(R3), C(R3)=NH, NH=NH;
B is C(R3), N(R2);
D is C(R3), N(R2);
where at least one of the members A, B or D must be nitrogen;
n are each independently 1 or 2;
L is a bond, -C(=O)-, -C(=S)-, -C(=O)-N(R2)-, -C(=O)-O-, -S(O)₀₋₂-, -S(O)₀₋₂-N(R2)-, a mono- or bicyclic ring system in which one or more ring members may be N(R3), O, S or -C(=O)-;
M is -C(=O)-N(R2)-, -N(R2)-C(=O)-, -S(O)₀₋₂-, -S(O)₀₋₂-N(R2)-, -N(R2)-S(O)₀₋₂-, -C(=O)-O-, -O-C(=O)-;
where the term "cycloalkyl" in each case represents a saturated or partly unsaturated ring
system which contains exclusively carbon atoms and which comprises one or more rings
and the physiologically compatible salts thereof.

2. A compound of the formula I as claimed in claim 1, wherein
R is hydrogen, (C₁-C₁₆)-alkyl, (C₁-C₅)-alkyloxy, (C₁-C₅)-alkylthio, (C₁-C₅)- alkylamino, di-(C₂-C₈)-alkylamino, (C₀-C₄)-alkylene-(C₆-C₁₀)-aryl, (C₀-C₄)- alkylene-(C₅-C₁₂)-heteroaryl, (C₀-C₄)-alkylene-(C₃-C₁₂)-heterocyclyl, (C₀-C₄)- alkylene-(C₃-C₁₂)-cycloalkyl, a bicyclic (C₈-C₁₄) ring system, where aryl, heteroaryl, heterocyclyl, cycloalkyl or the bicyclic (C₈-C₁₄) ring system may be mono- or polysubstituted by halogen, (C₁-C₆)-alkyl, (C₁-C₃)-alkyloxy, hydroxyl, (C₁-C₆)-alkylmercapto, amino, (C₁-C₆)- alkylamino, di-(C₂-C₁₂)-alkylamino, mono-(C₁-C₆)-alkylamino- carbonyl, di-(C₂-C₈)-alkylaminocarbonyl, (C₁-C₆)-alkoxycarbonyl, (C₁-C₆)-alkylcarbonyl, cyano, trifluoromethyl, trifluoromethyloxy, (C₁-C₆)-alkylsulfonyl or aminosulfonyl;
R1 is hydrogen, (C₁-C₁₀)-alkyl, (C₁-C₁₀)-alkyloxy, amino, mono-(C₁-C₁₀)-alkyl- amino, di-(C₂-C₁₂)-alkylamino, where alkyl may be substituted by halogen, (C₁-C₆)-alkyl, (C₁-C₃)- alkyloxy, hydroxyl, (C₁-C₆)-alkylmercapto, amino, (C₁-C₆)-alkylamino, di-(C₂-C₁₂)-alkylamino, -(C₆-C₁₀)-aryl, -(C₅-C₁₂)-heteroaryl, -(C₃-C₁₂)- heterocyclyl or -(C₃-C₁₂)-cycloalkyl, where aryl, heteroaryl, heterocyclyl or cycloalkyl may each optionally be mono- or polysubstituted by halogen, (C₁-C₆)- alkyl, (C₁-C₃)-alkyloxy, hydroxyl, (C₁-C₆)-alkylmercapto, amino, (C₁-C₆)-alkylamino, di-(C₂-C₁₂)-alkylamino;
R2 is hydrogen, (C₁-C₁₆)-alkyl, (C₀-C₄)-alkylene-(C₆-C₁₀)-aryl;
R3 is hydrogen, (C₁-C₆)-alkyl, (C₁-C₃)-alkyloxy, hydroxyl, (C₁-C₆)-alkylmercapto, amino, (C₁-C₆)-alkylamino, di-(C₂-C₁₂)-alkylamino, cyano, (C₁-C₆)-alkyl- carbonyl, halogen, trifluoromethyl, trifluoromethyloxy, (C₁-C₆)-alkylsulfonyl, aminosulfonyl;
A is O, S, N(R2), C(R3), C(R3)=C(R3);
B is C(R3), N(R2);
D is C(R3), N(R2);
where at least one of the members A, B or D must be nitrogen;
n are each independently 1 or 2;
L is a bond, -C(=O)-, -C(=S)-, -C(=O)-N(R2)-, -C(=O)-O-, -S(O)₀₋₂-, -S(O)₀₋₂-N(R2)-, a mono- or bicyclic ring system in which one or more ring members may be N(R3), O, S or -C(=O)-;
M is -C(=O)-N(R2)-, -N(R2)-C(=O)-, -S(O)₀₋₂-, -S(O)₀₋₂-N(R2)-, -N(R2)-S(O)₀₋₂-, -C(=O)-O-, -O-C(=O)-;
and the physiologically compatible salts thereof.

3. A compound of the formula I as claimed in claim 1 or 2, wherein
n is 1;
R is hydrogen, (C₁-C₁₆)-alkyl, (C₁-C₅)-alkyloxy, (C₁-C₅)-alkylthio, (C₁-C₅)-alkyl- amino, di-(C₂-C₈)-alkylamino, (C₀-C₄)-alkylene-(C₆-C₁₀)-aryl, (C₀-C₄)- alkylene-(C₅-C₁₂)-heteroaryl, (C₀-C₄)-alkylene-(C₃-C₁₂)-heterocyclyl, (C₀-C₄)- alkylene-(C₃-C₁₂)-cycloalkyl, a bicyclic (C₈-C₁₄) ring system, where aryl, heteroaryl, heterocyclyl, cycloalkyl or the bicyclic (C₈-C₁₄) ring system may be mono- or polysubstituted by halogen, (C₁-C₆)-alkyl, (C₁-C₃)-alkyloxy, hydroxyl, (C₁-C₆)-alkylmercapto, amino, (C₁-C₆)- alkylamino, di-(C₂-C₁₂)-alkylamino, mono-(C₁-C₆)-alkylamino- carbonyl, di -(C₂-C₈)-alkylaminocarbonyl, (C₁-C₆)-alkoxycarbonyl, (C₁-C₆)-alkylcarbonyl, cyano, trifluoromethyl, trifluoromethyloxy, (C₁-C₆)-alkylsulfonyl or aminosulfonyl;
R1 is (C₁-C₁₀)-alkyl, (C₁-C₁₀)-alkyloxy, amino, mono-(C₁-C₁₀)-alkylamino, di- (C₂-C₁₂)-alkylamino, where alkyl may be substituted by halogen, (C₁-C₆)-alkyl, (C₁-C₃)- alkyloxy, hydroxyl, (C₁-C₆)-alkylmercapto, amino, (C₁-C₆)-alkylamino, di-(C₂-C₁₂)-alkylamino, -(C₆-C₁₀)-aryl, -(C₅-C₁₂)-heteroaryl, -(C₃-C₁₂)- heterocyclyl or -(C₃-C₁₂)-cycloalkyl, where aryl, heteroaryl, heterocyclyl or cycloalkyl may each optionally be mono- or polysubstituted by halogen, (C₁-C₆)- alkyl, (C₁-C₃)-alkyloxy, hydroxyl, (C₁-C₆)-alkylmercapto, amino, (C₁-C₆)-alkylamino, di-(C₂-C₁₂)-alkylamino;
R2 is hydrogen, (C₁-C₁₆)-alkyl, (C₀-C₄)-alkylene-(C₆-C₁₀)-aryl;
R3 is hydrogen, (C₁-C₆)-alkyl, (C₁-C₃)-alkyloxy, hydroxyl, (C₁-C₆)-alkylmercapto, amino, (C₁-C₆)-alkylamino, di-(C₂-C₁₂)-alkylamino, cyano, (C₁-C₆)- alkylcarbonyl, halogen, trifluoromethyl, trifluoromethyloxy, (C₁-C₆)- alkylsulfonyl, aminosulfonyl;
A is O, S, N(R2), C(R3), C(R3)=C(R3);
B is C(R3), N(R2);
D is C(R3), N(R2);
where at least one of the members A, B or D must be nitrogen;
L is a bond, -C(=O)-, -C(=S)-, -C(=O)-N(R2)-, -C(=O)-O-, -S(O)₀₋₂-, -S(O)₀₋₂-N(R2)-, a mono- or bicyclic ring system in which one or more ring members may be N(R3), O, S or -C(=O)-;
M is -C(=O)-N(R2)-, -N(R2)-C(=O)-, -S(O)₀₋₂-, -S(O)₀₋₂-N(R2)-, -N(R2)-S(O)₀₋₂-, -O-C(=O)-;
and the physiologically compatible salts thereof.

4. A compound of the formula I as claimed in one or more of claims 1 to 3, wherein
n is 1;
R is hydrogen, (C₁-C₁₆)-alkyl, (C₁-C₅)-alkyloxy, (C₁-C₅)-alkylthio, (C₁-C₅)- alkylamino, di-(C₂-C₈)-alkylamino, (C₀-C₄)-alkylene-(C₆-C₁₀)-aryl, (C₀-C₄)- alkylene-(C₅-C₁₂)-heteroaryl, (C₀-C₄)-alkylene-(C₃-C₁₂)-heterocyclyl, (C₀-C₄)- alkylene-(C₃-C₁₂)-cycloalkyl, a bicyclic (C₈-C₁₄) ring system, where aryl, heteroaryl, heterocyclyl, cycloalkyl or the bicyclic (C₈-C₁₄) ring system may be mono- or polysubstituted by halogen, (C₁-C₆)-alkyl, (C₁-C₃)-alkyloxy, hydroxyl, (C₁-C₆)-alkylmercapto, amino, (C₁-C₆)- alkylamino, di-(C₂-C₁₂)-alkylamino, mono-(C₁-C₆)-alkylamino- carbonyl, di-(C₂-C₈)-alkylaminocarbonyl, (C₁-C₆)-alkoxycarbonyl, (C₁-C₆)-alkylcarbonyl, cyano, trifluoromethyl, trifluoromethyloxy,
R1 (C₁-C₆)-alkylsulfonyl or aminosulfonyl; is (C₁-C₁₀)-alkyl, (C₁-C₁₀)-alkyloxy, amino, mono-(C₁-C₁₀)-alkylamino, di- (C₂-C₁₂)-alkylamino, where alkyl may be substituted by halogen, (C₁-C₆)-alkyl, (C₁-C₃)- alkyloxy, hydroxyl, (C₁-C₆)-alkylmercapto, amino, (C₁-C₆)-alkylamino, di-(C₂-C₁₂)-alkylamino, -(C₆-C₁₀)-aryl, -(C₅-C₁₂)-heteroaryl, -(C₃-C₁₂)- heterocyclyl or -(C₃-C₁₂)-cycloalkyl, where aryl, heteroaryl, heterocyclyl or cycloalkyl may each optionally be mono- or polysubstituted by halogen, (C₁-C₆)- alkyl, (C₁-C₃)-alkyloxy, hydroxyl, (C₁-C₆)-alkylmercapto, amino, (C₁-C₆)-alkylamino, di-(C₂-C₁₂)-alkylamino;
R2 is hydrogen, (C₁-C₁₆)-alkyl, (C₀-C₄)-alkylene-(C₆-C₁₀)-aryl;
R3 is hydrogen, (C₁-C₆)-alkyl, (C₁-C₃)-alkyloxy, hydroxyl, (C₁-C₆)-alkylmercapto, amino, (C₁-C₆)-alkylamino, di-(C₂-C₁₂)-alkylamino, cyano, (C₁-C₆)- alkylcarbonyl, halogen, trifluoromethyl, trifluoromethyloxy, (C₁-C₆)- alkylsulfonyl, aminosulfonyl;
A is S, C(R3)=C(R3);
B is C(R3);
D is N(R2);
L is a bond, -C(=O)-, -C(=S)-, -C(=O)-N(R2)-, -C(=O)-O-, -S(O)₀₋₂-, -S(O)₀₋₂-N(R2)-, a mono- or bicyclic ring system in which one or more ring members may be N(R3), O, S or -C(=O)-;
M is -C(=O)-N(R2)-, -N(R2)-C(=O)-, -S(O)₀₋₂-, -S(O)₀₋₂-N(R2)-, -N(R2)-S(O)₀₋₂-, -O-C(=O)-;
and the physiologically compatible salts thereof.

5. A compound of the formula I as claimed in one or more of claims 1 to 4, wherein
n is 1;
R is (C₁-C₁₆)-alkyl, (C₁-C₅)-alkyloxy, (C₀-C₄)-alkylene-(C₆-C₁₀)-aryl, a bicyclic (C₈-C₁₄) ring system, where aryl or the bicyclic (C₈-C₁₄) ring system may be mono- or polysubstituted by halogen, (C₁-C₆)-alkyl, (C₁-C₃)-alkyloxy, hydroxyl, (C₁-C₆)-alkylmercapto, amino, (C₁-C₆)-alkylamino, di-(C₂-C₁₂)- alkylamino, mono-(C₁-C₆)-alkylaminocarbonyl, di-(C₂-C₈)-alkyl- aminocarbonyl, (C₁-C₆)-alkoxycarbonyl, (C₁-C₆)-alkylcarbonyl, cyano, trifluoromethyl, trifluoromethyloxy, (C₁-C₆)-alkylsulfonyl or aminosulfonyl;
R1 is (C₁-C₁₀)-alkyl, (C₁-C₁₀)-alkyloxy, amino, mono-(C₁-C₁₀)-alkylamino, di- (C₂-C₁₂)-alkylamino, where alkyl may be substituted by halogen, (C₁-C₆)-alkyl, (C₁-C₃)- alkyloxy, hydroxyl, (C₁-C₆)-alkylmercapto, amino, (C₁-C₆)-alkylamino, di-(C₂-C₁₂)-alkylamino, -(C₆-C₁₀)-aryl, -(C₅-C₁₂)-heteroaryl, -(C₃-C₁₂)- heterocyclyl or -(C₃-C₁₂)-cycloalkyl, where aryl, heteroaryl, heterocyclyl or cycloalkyl may each optionally be mono- or polysubstituted by halogen, (C₁-C₆)₋ alkyl, (C₁-C₃)-alkyloxy, hydroxyl, (C₁-C₆)-alkylmercapto, amino, (C₁-C₆)-alkylamino, di-(C₂-C₁₂)-alkylamino;
R2 is hydrogen, (C₁-C₁₆)-alkyl, (C₀-C₄)-alkylene-(C₆-C₁₀)-aryl;
R3 is hydrogen, (C₁-C₆)-alkyl, (C₁-C₃)-alkyloxy, hydroxyl, (C₁-C₆)-alkylmercapto, amino, (C₁-C₆)-alkylamino, di-(C₂-C₁₂)-alkylamino, cyano, (C₁-C₆)-alkyl- carbonyl, halogen, trifluoromethyl, trifluoromethyloxy, (C₁-C₆)-alkylsulfonyl, aminosulfonyl;
A is S, C(R3)=C(R3);
B is C(R3);
D is N(R2);
L is a bond, -C(=O)-;
M is -C(=O)-N(R2)-, -O-C(=O)-;
and the physiologically compatible salts thereof.

6. A compound as claimed in one or more of claims 1 to 5 for use as a medicament.

7. A medicament comprising one or more compounds as claimed in one or more of claims 1 to 5.

8. A medicament comprising one or more compounds as claimed in one or more of claims 1 to 5 and at least one further active ingredient.

9. The medicament as claimed in claim 8, which comprises, as a further active ingredient, one or more antidiabetics, active hypoglycemic ingredients, HMG-CoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, MTP inhibitors, CETP inhibitors, polymeric bile acid adsorbers, LDL receptor inducers, ACAT inhibitors, antioxidants, lipoprotein lipase inhibitors, ATP citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, α-glucosidase inhibitors, active ingredients which act on the ATP-dependent potassium channel of the beta cells, glycogen phosphorylase inhibitors, glucagon receptor antagonists, activators of glucokinase, inhibitors of gluconeogenesis, inhibitors of fructose 1,6-biphosphatase, modulators of glucose transporter 4, inhibitors of glutamine:fructose-6-phosphate amidotransferase, inhibitors of dipeptidylpeptidase IV, inhibitors of 11-beta-hydroxysteroid dehydrogenase 1, inhibitors of protein tyrosine phosphatase 1B, modulators of the sodium-dependent glucose transporter 1 or 2, modulators of GPR40, inhibitors of hormone-sensitive lipase, inhibitors of acetyl-CoA carboxylase, inhibitors of phosphoenolpyruvate carboxykinase, inhibitors of glycogen synthase kinase-3 beta, inhibitors of protein kinase C beta, endothelin-A receptor antagonists, inhibitors of I kappaB kinase, modulators of the glucocorticoid receptor, CART agonists, NPY agonists, MC4 agonists, orexin agonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, decoupling protein 2 or 3 modulators, leptin agonists, DA agonists (bromocriptin, doprexin), lipase/amylase inhibitors, PPAR modulators, RXR modulators or TR-β-agonists or amphetamines.

10. The use of the compounds as claimed in one or more of claims 1 to 5 for producing a medicament for treatment of diabetes.

11. The use of the compounds as claimed in one or more of claims 1 to 5 for producing a medicament for reducing lipids.

12. The use of the compounds as claimed in one or more of claims 1 to 5 for producing a medicament for treatment of metabolic syndrome.

13. The use of the compounds as claimed in one or more of claims 1 to 5 for producing a medicament for treatment of insulin resistance.

14. The use of the compounds as claimed in one or more of claims 1 to 5 for producing a medicament for treatment of obesity.

15. The use of the compounds as claimed in one or more of claims 1 to 5 for producing a medicament for treatment of cardiovascular disorders.

16. The use of the compounds as claimed in one or more of claims 1 to 5 for producing a medicament for treatment of CNS disorders.

17. The use of the compounds as claimed in one or more of claims 1 to 5 for producing a medicament for treatment of schizophrenia.

18. The use of the compounds as claimed in one or more of claims 1 to 5 for producing a medicament for treatment of Alzheimer's.

19. A process for producing a medicament comprising one or more of the compounds as claimed in one or more of claims 1 to 5, which comprises mixing the active ingredient with a pharmaceutically suitable carrier and bringing this mixture into a form suitable for administration.

## Revendications

1. Composés de formule I, 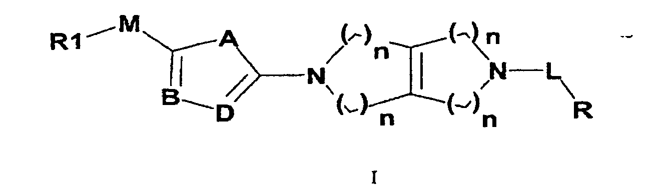 où
R signifie hydrogène, (C₁-C₁₆)-alkyle, (C₁-C₅)- alkyloxy, (C₁-C₅)-alkylthio, (C₁-C₅)-alkylamino, di-(C₂-C₈)-alkylamino, (C₀-C₄)-alkylène-(C₆-C₁₀)- aryle, (C₀-C₄)-alkylène-(C₅-C₁₂)-hétéroaryle, (C₀- C₄)-alkylène-(C₃-C₁₂)-hétérocyclyle, (C₀-C₄)- alkylène-(C₃-C₁₂)-cycloalkyle, un système bicyclique en (C₈-C₁₄), où aryle, hétéroaryle, hétérocyclyle cycloalkyle ou le système bicyclique en (C₈- C₁₄) peuvent être monosubstitués ou polysubstitués par halogène, (C₁-C₆)-alkyle, (C₁-C₃)-alkyloxy, hydroxy, (C₁-C₆)- alkylmercapto, amino, (C₁-C₆)-alkylamino, di- (C₂-C₁₂) -alkylamino, mono-(C₁-C₆)- alkylaminocarbonyle, di-(C₂-C₈)- alkylaminocarbonyle, (C₁-C₆)-alcoxycarbonyle, (C₁-C₆)-alkylcarbonyle, cyano, trifluorométhyle, trifluorométhyloxy, (C₁-C₆)- alkylsulfonyle ou aminosulfonyle ;
R1 signifie hydrogène, (C₁-C₁₀)-alkyle, (C₁-C₁₀)- alkyloxy, amino, mono-(C₁-C₁₀)-alkylamino, di-(C₂- C₁₂)-alkylamino, où alkyle peut être substitué par halogène, (C₁-C₆)-alkyle, (C₁-C₃)-alkyloxy, hydroxy, (C₁- C₆)-alkylmercapto, amino, (C₁-C₆)-alkylamino, di-(C₂-C₁₂)-alkylamino, -(C₆-C₁₀)-aryle, -(C₅- C₁₂)-hétéroaryle, -(C₃-C₁₂)-hétérocyclyle ou -(C₃-C₁₂)-cycloalkyle, où aryle, hétéroaryle, hétérocyclyle ou cycloalkyle peuvent le cas échéant être monosubstitués ou polysubstitués par halogène, (C₁-C₆)-alkyle, (C₁-C₃)- alkyloxy, hydroxy, (C₁-C₆)-alkylmercapto, amino, (C₁-C₆)-alkylamino, di-(C₂-C₁₂)- alkylamino ;
R2 signifie hydrogène, (C₁-C₁₆)-alkyle, (C₀-C₄)- alkylène-(C₆-C₁₀)-aryle;
R3 signifie hydrogène, (C₁-C₆)-alkyle, (C₁-C₃)- alkyloxy, hydroxy, (C₁-C₆)-alkylmercapto, amino, (C₁-C₆)-alkylamino, di-(C₂-C₁₂)-alkylamino, cyano, (C₁-C₆)-alkylcarbonyle, halogène, trifluorométhyle, trifluorométhyloxy, (C₁-C₆)-alkylsulfonyle, aminosulfonyle ;
A signifie O, S, N(R2), C(R3), C(R3)=C(R3), NH=C(R3), C(R3)=NH, NH=NH ;
B signifie C(R3), N(R2) ;
D signifie C(R3), N(R2) ; où au moins un des chaînons A, B ou D doit représenter azote ;
n indépendamment l'un de l'autre, valent à chaque fois 1 ou 2 ;
L signifie une liaison, -C(=O)-, -C(=S)-, -C(=O)- N(R2)-, -C(=O)-O-, -S(O)₀₋₂-, -S(O)₀₋₂-N(R2)-, un système monocyclique ou bicyclique, où un ou plusieurs chaînons de cycle peuvent être N(R3), O, S ou -C(=O)- ;
M signifie -C(=O)-N(R2)-, -N(R2)-C(=O)-, -S(O)₀₋₂-, -S(O)₀₋₂-N(R2)-, -N(R2)-S(O)₀₋₂-, -C(=O)-O-, -O- C(=O)- ; où le concept "cycloalkyle" représente à chaque fois un système cyclique saturé ou partiellement insaturé, qui contient exclusivement des atomes de carbone et qui comprend un ou plusieurs cycles
et leurs sels physiologiquement acceptables.

2. Composés de formule I selon la revendication 1, **caractérisés en ce que**
R signifie hydrogène, (C₁-C₁₆)-alkyle, (C₁-C₅)- alkyloxy, (C₁-C₅)-alkylthio, (C₁-C₅)-alkylamino, di-(C₂-C₈)-alkylamino, (C₀-C₄)-alkylène-(C₆-C₁₀)- aryle, (C₀-C₄)-alkylène-(C₅-C₁₂)-hétéroaryle, (C₀- C₄)-alkylène-(C₃-C₁₂)-hétérocyclyle, (C₀-C₄)- alkylène-(C₃-C₁₂)-cycloalkyle, un système bicyclique en (C₈-C₁₄, où aryle, hétéroaryle, hétérocyclyle cycloalkyle ou le système bicyclique en (C₈- C₁₄) peuvent être monosubstitués ou polysubstitués par halogène, (C₁-C₆)-alkyle, (C₁-C₃)-alkyloxy, hydroxy, (C₁-C₆)- alkylmercapto, amino, (C₁-C₆)-alkylamino, di- (C₂-C₁₂)-alkylamino, mono-(C₁-C₆)- alkylaminocarbonyle, di-(C₂-C₈)- alkylaminocarbonyle, (C₂-C₆)-alcoxycarbonyle, (C₁-C₆)-alkylcarbonyle, cyano, trifluorométhyle, trifluorométhyloxy, (C₁-C₆)- alkylsulfonyle ou aminosulfonyle ;
R1 signifie hydrogène, (C₁-C₁₀)-alkyle, (C₁-C₁₀)- alkyloxy, amino, mono-(C₁-C₁₀)-alkylamino, di-(C₂- C₁₂)-alkylamino, où alkyle peut être substitué par halogène, (C₁-C₆)-alkyle, (C₁-C₃)-alkyloxy, hydroxy, (C₁- C₆)-alkylmercapto, amino, (C₁-C₆)-alkylamino, di-(C₂-C₁₂)-alkylamino, -(C₆-C₁₀)-aryle, -(C₅- C₁₂)-hétéroaryle, -(C₃-C₁₂)-hétérocyclyle ou -(C₃-C₁₂)-cycloalkyle, où aryle, hétéroaryle, hétérocyclyle ou cycloalkyle peuvent le cas échéant être monosubstitués ou polysubstitués par halogène, (C₁-C₆)-alkyle, (C₁-C₃)-alkyloxy, hydroxy, (C₁-C₆)-alkylmercapto, amino, (C₁- C₆)-alkylamino, di-(C₂-C₁₂)-alkylamino;
R2 signifie hydrogène, (C₁-C₁₆)-alkyle, (C₀-C₄)- alkylène-(C₆-C₁₀)-aryle ;
R3 signifie hydrogène, (C₁-C₆)-alkyle, (C₁-C₃)- alkyloxy, hydroxy, (C₁-C₆)-alkylmercapto, amino, (C₁-C₆)-alkylamino, di-(C₂-C₁₂)-alkylamino, cyano, (C₁-C₆)-alkylcarbonyle, halogène, trifluorométhyle, trifluorométhyloxy, (C₁-C₆)-alkylsulfonyle, aminosulfonyle ;
A signifie 0, S, N(R2), C(R3), C(R3)=C(R3) ;
B signifie C(R3), N(R2) ;
D signifie C(R3), N(R2) ; où au moins un des chaînons A, B ou D doit représenter azote ;
n indépendamment l'un de l'autre, valent à chaque fois 1 ou 2 ;
L signifie une liaison, -C(=O)-, -C(=S)-, -C(=O)- N(R2)-, -C(=O)-O-, -S(O)₀₋₂-, -S(O)₀₋₂-N(R2)-, un système monocyclique ou bicyclique, où un ou plusieurs chaînons de cycle peuvent être N(R3), O, S ou -C(=O)- ;
M signifie -C(=O)-N(R2)-, -N(R2)-C(=O)-, -S(O)₀₋₂-, -S(O)₀₋₂-N(R2)-, -N(R2)-S(O)₀₋₂, -C(=O)-O-, -O- C(=O)- ;
et leurs sels physiologiquement acceptables.

3. Composés de formule I selon la revendication 1 ou 2, **caractérisés en ce que**
n vaut 1 ;
R signifie hydrogène, (C₁-C₁₆)-alkyle, (C₁-C₅)- alkyloxy, (C₁-C₅)-alkylthio, (C₁-C₅)-alkylamino, di-(C₂-C₈)-alkylamino, (C₀-C₄)-alkylène-(C₆-C₁₀- aryle, (C₀-C₄)-alkylène-(C₅-C₁₂)-hétéroaryle, (C₀- C₄)-alkylène-(C₃-C₁₂)-hétérocyclyle, (C₀-C₄)- alkylène-(C₃-C₁₂)-cycloalkyle, un système bicyclique en (C₈-C₁₄), où aryle, hétéroaryle, hétérocyclyle, cycloalkyle ou le système bicyclique en (C₈- C₁₄) peuvent être monosubstitués ou polysubstitués par halogène, (C₁-C₆)-alkyle, (C₁-C₃)-alkyloxy, hydroxy, (C₁-C₆)- alkylmercapto, amino, (C₁-C₆)-alkylamino, di- (C₂-C₁₂)-alkylamino, mono-(C₁-C₆)- alkylaminocarbonyle, di-(C₂-C₈)- alkylaminocarbonyle, (C₁-C₆)-alcoxycarbonyle, (C₁-C₆)-alkylcarbonyle, cyano, trifluorométhyle, trifluorométhyloxy, (C₁-C₆)- alkylsulfonyle ou aminosulfonyle ;
R1 signifie (C₁-C₁₀)-alkyle, (C₁-C₁₀)-alkyloxy, amino, mono-(C₁-C₁₀)-alkylamino, di-(C₂-C₁₂)-alkylamino, où alkyle peut être substitué par halogène, (C₁-C₆)-alkyle, (C₁-C₃)-alkyloxy, hydroxy, (C₁- C₆)-alkylmercapto, amino, (C₁-C₆)-alkylamino, di-(C₂-C₁₂)-alkylamino, -(C₆-C₁₀)-aryle, -(C₅- C₁₂)-hétéroaryle, -(C₃-C₁₂)-hétérocyclyle ou -(C₃-C₁₂)-cycloalkyle, où aryle, hétéroaryle, hétérocyclyle ou cycloalkyle peuvent le cas échéant être monosubstitués ou polysubstitués par halogène, (C₁-C₆)-alkyle, (C₁-C₃)- alkyloxy, hydroxy, (C₁-C₆)-alkylmercapto, amino, (C₁-C₆)-alkylamino, di-(C₂-C₁₂)- alkylamino ;
R2 signifie hydrogène, (C₁-C₁₆)-alkyle, (C₀-C₄)- alkylène-(C₆-C₁₀)-aryle ;
R3 signifie hydrogène, (C₁-C₆)-alkyle, (C₁-C₃)- alkyloxy, hydroxy, (C₁-C₆)-alkylmercapto, amino, (C₁-C₆)-alkylamino, di-(C₂-C₁₂)-alkylamino, cyano, (C₁-C₆)-alkylcarbonyle, halogène, trifluorométhyle, trifluorométhyloxy, (C₁-C₆)-alkylsulfonyle, aminosulfonyle ;
A signifie O, S, N(R2), C(R3), C(R3)=C(R3) ;
B signifie C(R3), N(R2) ;
D signifie C(R3), N(R2) ;
où au moins un des chaînons A, B ou D doit représenter azote ;
L signifie une liaison, -C(=O)-, -C(=S)-, -C(=O)- N(R2)-, -C(=O)-O-, -S(O)₀₋₂-, -S(O)₀₋₂-N(R2)-, un système monocyclique ou bicyclique, où un ou plusieurs chaînons de cycle peuvent être N(R3), O, S ou -C(=O)- ;
M signifie -C(=O)-N(R2)-, N(R2)-C(=O)-, -S(O)₀₋₂-, -S(O)₀₋₂N(R2)-, -N(R2)-S(O)₀₋₂-, -O-C(=O)- ;
et leurs sels physiologiquement acceptables.

4. Composés de formule I selon l'une ou plusieurs des revendications 1 à 3, **caractérisés en ce que**
n vaut 1 ;
R signifie hydrogène, (C₁-C₁₆)-alkyle, (C₁-C₅)- alkyloxy, (C₁-C₅)-alkylthio, (C₁-C₅)-alkylamino, di-(C₂-C₈)-alkylamino, (C₀-C₄)-alkylène-(C₆-C₁₀)- aryle, (C₀-C₄)-alkylène-(C₅-C₁₂)-hétéroaryle, (C₀- C₄)-alkylène-(C₃-C₁₂)-hétérocyclyle, (C₀-C₄)- alkylène-(C₃-C₁₂)-cycloalkyle, un système bicyclique en (C₈-C₁₄), où aryle, hétéroaryle, hétérocyclyle, cycloalkyle ou le système bicyclique en (C₈- C₁₄) peuvent être monosubstitués ou polysubstitués par halogène, (C₁-C₆)-alkyle, (C₁-C₃)-alkyloxy, hydroxy, (C₁-C₆)- alkylmercapto, amino, (C₁-C₆)-alkylamino, di- (C₂-C₁₂)-alkylamino, mono-(C₁-C₆)- alkylaminocarbonyle, di-(C₂-C₈)- alkylaminocarbonyle, (C₁-C₆)-alcoxycarbonyle, (C₁-C₆)-alkylcarbonyle, cyano, trifluorométhyle, trifluorométhyloxy, (C₁-C₆)- alkylsulfonyle ou aminosulfonyle ;
R1 signifie (C₁-C₁₀)-alkyle, (C₁-C₁₀)-alkyloxy, amino, Mono-(C₁-C₁₀)-alkylamino, di-(C₂-C₁₂)-alkylamino, où alkyle peut être substitué par halogène, (C₁-C₆)-alkyle, (C₁-C₃)-alkyloxy, hydroxy, (C₁- C₆)-alkylmercapto, amino, (C₁-C₆)-alkylamino, di-(C₂-C₁₂)-alkylamino, -(C₆-C₁₀)-aryle, -(C₅- C₁₂)-hétéroaryle, -(C₃-C₁₂)-hétérocyclyle ou -(C₃-C₁₂)-cycloalkyle, où aryle, hétéroaryle, hétérocyclyle ou cycloalkyle peuvent le cas échéant être monosubstitués ou polysubstitués par halogène, (C₁-C₆)-alkyle, (C₁-C₃)- alkyloxy, hydroxy, (C₁-C₆)-alkylmercapto, amino, (C₁-C₆)-alkylamino, di-(C₂-C₁₂)- alkylamino ;
R2 signifie hydrogène, (C₁-C₁₆)-alkyle, (C₀-C₄)- alkylène-(C₆-C₁₀)-aryle ;
R3 signifie hydrogène, (C₁-C₆)-alkyle, (C₁-C₃)- alkyloxy, hydroxy, (C₁-C₆)-alkylmercapto, amino, (C₁-C₆)-alkylamino, di-(C₂-C₁₂)-alkylamino, cyano, (C₁-C₆)-alkylcarbonyle, halogène, trifluorométhyle, trifluorométhyloxy, (C₁-C₆)-alkylsulfonyle, aminosulfonyle ;
A signifie S, C(R3)=C(R3) ;
B signifie C(R3) ;
D signifie N(R2) ;
L signifie une liaison, -C(=O)-, -C(=S)-, -C(=O)- N(R2)-, -C(=O)-O-, -S(O)₀₋₂-, -S(O)₀₋₂-N(R2)-, un système monocyclique ou bicyclique, où un ou plusieurs chaînons de cycle peuvent être N(R3), O, S ou -C(=O)- ;
M signifie -C(=O)-N(R2)-, N(R2)-C(=O)-, -S(O)₀₋₂-, -S(O)₀₋₂-N(R2)-, -N(R2)S(O)₀₋₂-, -O-C(=O)- ;
et leurs sels physiologiquement acceptables.

5. Composés de formule I selon l'une ou plusieurs des revendications 1 à 4, **caractérisés en ce que** n    vaut 1 ;
R signifie (C₁-C₁₆)-alkyle, (C₁-C₅)-alkyloxy, (C₀-C₄)- alkylène-(C₆-C₁₀)-aryle, un système bicyclique en (C₈-C₁₄), où aryle ou le système bicyclique en (C₈-C₁₄) peuvent être monosubstitués ou polysubstitués par halogène, (C₁-C₆)-alkyle, (C₁-C₃)- alkyloxy, hydroxy, (C₁-C₆)-alkylmercapto, amino, (C₁-C₆)-alkylamino, di-(C₂-C₁₂)- alkylamino, mono-(C₁-C₆)-alkylaminocarbonyle, di-(C₂-C₈)-alkylaminocarbonyle, (C₁-C₆)- alcoxycarbonyle, (C₁-C₆)-alkylcarbonyle, cyano, trifluorométhyle, trifluorométhyloxy, (C₁-C₆)-alkylsulfonyle ou aminosulfonyle ;
R1 signifie -(C₁-C₁₀)-alkyle, (C₁-C₁₀)-alkyloxy, amino, Mono-(C₁-C₁₀)-alkylamino, di-(C₂-C₁₂)-alkylamino, où alkyle peut être substitué par halogène, (C₁-C₆)-alkyle, (C₁-C₃)-alkyloxy, hydroxy, (C₁- C₆)-alkylmercapto, amino, (C₁-C₆)-alkylamino, di-(C₂-C₁₂)-alkylamino, -(C₆-C₁₀)-aryle, -(C₅- C₁₂)-hétéroaryle, -(C₃-C₁₂)-hétérocyclyle ou -(C₃-C₁₂)-cycloalkyle, où aryle, hétéroaryle, hétérocyclyle ou cycloalkyle peuvent le cas échéant être monosubstitués ou polysubstitués par halogène, (C₁-C₆)-alkyle, (C₁-C₃)-alkyloxy, hydroxy, (C₁-C₆)-alkylmercapto, amino, (C₁- C₆)-alkylamino, di-(C₂-C₁₂)-alkylamino ;
R2 signifie hydrogène, (C₁-C₁₆)-alkyle, (C₀-C₄)- alkylène-(C₆-C₁₀)-aryle ;
R3 signifie hydrogène, (C₁-C₆)-alkyle, (C₁-C₃)- alkyloxy, hydroxy, (C₁-C₆)-alkylmercapto, amino, (C₁-C₆)-alkylamino, di-(C₂-C₁₂)-alkylamino, cyano, (C₁-C₆)-alkylcarbonyle, halogène, trifluorométhyle, trifluorométhyloxy, (C₁-C₆)-alkylsulfonyle, aminosulfonyle ;
A signifie S, C(R3)=C(R3) ;
B signifie C(R3) ;
D signifie N(R2) ;
L signifie une liaison, -C(=O)- ;
M signifie -C(=O)-N(R2)-, -O-C(=O)- ; et leurs sels physiologiquement acceptables.

6. Composés selon l'une ou plusieurs des revendications 1 à 5 destinés à être utilisés comme médicament.

7. Médicament contenant un ou plusieurs des composés selon l'une ou plusieurs des revendications 1 à 5.

8. Médicament contenant un ou plusieurs des composés selon l'une ou plusieurs des revendications 1 à 5 et au moins une autre substance active.

9. Médicament selon la revendication 8, **caractérisé en ce qu'**il contient comme autre substance active un(e) ou plusieurs antidiabétiques, substances actives hypoglycémiques, inhibiteurs de la HMGCoA-réductase, inhibiteurs de la résorption de cholestérol, agonistes de PPAR gamma, agonistes de PPAR alpha, agonistes de PPAR alpha/gamma, agonistes de PPAR delta, fibrates, inhibiteurs de MTP, inhibiteurs de la résorption de l'acide biliaire, inhibiteurs de MTP, inhibiteurs de CETP, adsorbants polymères de l'acide biliaire, inducteurs de récepteurs de LDL, inhibiteurs d'ACAT, antioxydants, inhibiteurs de la lipoprotéine-lipase, inhibiteurs de l'ATP-citrate lyase, inhibiteurs de la squalène-synthétase, antagonistes de la lipoprotéine (a), agonistes du récepteur HM74A, inhibiteurs de lipase, insulines, sulfonylurées, biguanides, Méglitinides, thiazolidinediones, inhibiteurs de l'α-glucosidase, substances actives agissant sur le canal potassique dépendant d'ATP des cellules bêta, inhibiteurs de la glycogène phosphorylase, antagonistes du récepteur du glucagon, activateurs de la glucokinase, inhibiteurs de la gluconéogenèse, inhibiteurs de la fructose-1,6-biphosphatase, modulateurs du transporteur 4 du glucose, inhibiteurs de la glutamine-fructose-6-phosphate-amidotransférase, inhibiteurs de la dipeptidylpeptidase-IV, inhibiteurs de la 11-bêta-hydroxystéroïde-déshydrogénase-1, inhibiteurs de la protéine-tyrosine-phosphatase-1B, modulateurs du transporteur 1 ou 2 du glucose dépendant du sodium, modulateurs du GPR40, inhibiteurs de la lipase sensible aux hormones, inhibiteurs de l'acétyl-CoA carboxylase, inhibiteurs de la phosphoénol-pyruvate-carboxykinase, inhibiteurs de la glycogène synthase kinase-3 bêta, inhibiteurs de la protéine kinase C bêta, antagonistes du récepteur de l'endothéline-A, inhibiteurs de la I kappaB kinase, modulateurs du récepteur des glucocorticoïdes, agonistes de CART, agonistes de NPY, agonistes de MC4, agonistes d'orexine, agonistes de H3, agonistes de TNF, agonistes de CRF, antagonistes de CRF BP, agonistes d'urocortine, agonistes de β3, antagonistes du récepteur de CB1, agonistes de MSH (hormone stimulant les mélanocytes), agonistes de CCK, inhibiteurs de la résorption de la sérotonine, composés sérotoninergiques et noradrénergiques mixtes, agonistes de 5HT, agonistes de bombésine, agonistes de galanine, hormones de croissance, composés libérant l'hormone de croissance, agonistes de TRH, modulateurs 2 ou 3 de la protéine découplante, agonistes de leptine, agonistes de DA (Bromocriptine, Doprexine), inhibiteurs de lipase/amylase, modulateurs de PPAR, modulateurs de RXR, agonistes de TR-β ou amphétamines.

10. Utilisation des composés selon l'une ou plusieurs des revendications 1 à 5 pour la préparation d'un médicament destiné au traitement du diabète.

11. Utilisation des composés selon l'une ou plusieurs des revendications 1 à 5 pour la préparation d'un médicament destiné à abaisser les lipides.

12. Utilisation des composés selon l'une ou plusieurs des revendications 1 à 5 pour la préparation d'un médicament destiné au traitement du syndrome métabolique.

13. Utilisation des composés selon l'une ou plusieurs des revendications 1 à 5 pour la préparation d'un médicament destiné au traitement de la résistance à l'insuline.

14. Utilisation des composés selon l'une ou plusieurs des revendications 1 à 5 pour la préparation d'un médicament destiné au traitement de l'obésité.

15. Utilisation des composés selon l'une ou plusieurs des revendications 1 à 5 pour la préparation d'un médicament destiné au traitement de maladies cardiovasculaires.

16. Utilisation des composés selon l'une ou plusieurs des revendications 1 à 5 pour la préparation d'un médicament destiné au traitement de maladies du système nerveux central.

17. Utilisation des composés selon l'une ou plusieurs des revendications 1 à 5 pour la préparation d'un médicament destiné au traitement de la schizophrénie.

18. Utilisation des composés selon l'une ou plusieurs des revendications 1 à 5 pour la préparation d'un médicament destiné au traitement de la maladie d'Alzheimer.

19. Procédé pour la préparation d'un médicament contenant un ou plusieurs des composés selon l'une ou plusieurs des revendications 1 à 5, **caractérisé en ce que** la substance active est mélangée avec un support pharmaceutiquement approprié et ce mélange est amené dans une forme appropriée pour l'administration.
